# EUROPEAN PATENT APPLICATION

(11) **EP 4 190 521 A1**
(43) Date of publication of application: **07.06.2023**
(21) Application number: 21212132.1
(22) Date of filing: 03.12.2021
(51) Int. Cl.: B27L 1/00, B27L 1/10, G01N 33/46

(54) **A METHOD FOR MANUFACTURING VENEER**

(71) Applicant: UPM Plywood Oy, 15140 Lahti (FI)
(72) Inventor: Marttila, Heikki, 00100 Helsinki (FI); Salmikuukka, Timo, 00100 Helsinki (FI); Korhonen, Juha, 00100 Helsinki (FI); Sorjonen, Mika, 00100 Helsinki (FI); Uski, Juha, 00100 Helsinki (FI); Koponen, Simo, 00100 Helsinki (FI)
(74) Representative: Berggren Oy

(57) **Abstract**

A method for manufacturing veneer. The method comprises conditioning a first log or a first primary block cross-cut from the first log, debarking the first log or the first primary block using a debarking apparatus, and peeling the first log or the first primary block using a lathe to form the veneer. The method comprises before conditioning, debarking, and peeling the first log or the first primary block, imaging the first log a first time to determine first primary information of the first log, the first primary information being indicative of at least one of the following: (i) information of knots in the first log, (ii) density profile of the first log, and (iii) shape of the first log; storing the first primary information in a first database; after imaging the first log the first time and before peeling the first log or the first primary block, analysing the first log or the first primary block a second time to determine first secondary information; and accessing the first database with the first secondary information to receive the first primary information from the first database. The method comprises controlling the debarking apparatus and/or the lathe using the first primary information of the first log.

## Description

### Technical field

The invention relates to a method and an arrangement for manufacturing veneer, i.e. a sheet of wood. The veneer may be clipped and dried; and used for manufacturing laminate products including plywood and laminated veneer lumber. The invention relates to a method for manufacturing laminated wood boards including plywood and laminated veneer lumber.

### Background

Plywood or laminated veneer lumber (LVL) is manufactured by laying veneer sheets on top of each other, adhesive in between, and pressing them to form a panel, i.e. a plywood panel or an LVL panel. Such a panel may be sawn to smaller pieces according to needs. The veneer sheets are clipped from a large veneer web (hereinafter a veneer), and the veneer sheets and/or the veneer (i.e. veneer web) has been dried before pressing. Commonly veneer is manufactured from logs by peeling with a lathe.

As feedstock material the logs are heterogeneous. Their diameter, straightness, and internal structure including amount and position of knots and density profile may vary from log to log. This has the effect that different amounts of veneer usable for plywood or LVL manufacturing can be obtained from different logs. Moreover, some logs are not usable at all. Nowadays the logs are examined electromagnetically, and a log discarded if its quality is below a limit. The acceptable logs are processed all in a somewhat similar manner. This may result in the quality of the veneer being sub-optimal or even not acceptable, whereby also some veneer material is disposed, resulting in reduction of yield. Nowadays the quality of the veneer is examined optically, and such veneer is discarded of which quality is below a limit. In certain types of products, the veneer of highest quality is arranged as a surface layer of the product (LVL, plywood), while the middle layers may have lower-quality veneers. Even so, there may be a shortage of such veneer sheets that are acceptable as surface veneer sheets.

In addition, the logs are debarked before peeling. Debarking too little material from the logs results in problems in peeling, as bits of bark may generate peeling defects to the veneer, which lowers a yield of high quality veneer. Peeling defects include integrity of the veneer, presence and amount of cracks, wave-like surface of the veneer, projections and/or valleys on the veneer surface, thickness variation, and excessive surface roughness. However, debarking too much material from the logs sometimes results is usable material being removed from the logs at a too early stage, in this way lowering the yield. Debarking too hard may also reduce a quality of sapwood of the log, which reduces the quality of the veneer and may even result is lesser yield of such veneer that is acceptable as surface veneer.

### Summary

It has been found that the yield of high quality veneer can be improved in two ways: (1) by optimising the peeling log-wise so that an amount of discarded veneer material can be minimized, and conversely the yield maximized (concerning also the yield of the highest quality surface veneer); and/or (2) by optimising the debarking so that only the needed amount of bark is removed by the debarking. It has been found that both these optimizations may be based on primary information of the log, the primary information being indicative of at least one of the following: (i) information of knots in the log, (ii) density profile of the log, and (iii) shape of the log. Peeling can be optimized by controlling a lathe. Debarking can be controlled by controlling a debarking apparatus. As detailed below, another purpose of the primary information is to associate the primary information with a secondary information.

In addition, it has been found that the manufacturing process can be optimized by classifying the logs based on the primary information to different quality classes. One of the quality classes may correspond to so low quality that the log is not to be used, at least not to be used as such, for veneer manufacturing at all. As for the other logs, they may be stored in different storages according to their quality class before peeling. For classification, it is beneficial that the primary information being indicative of at least one of: (iv) absence of a foreign objects from the log, and (v) absence of decay from the log.

Measuring the primary information the log with such an accuracy that it can be used both for classifying the log and for optimizing the debarking and/or peeling needs reasonably expensive equipment. Moreover, during storing, the information may be lost; or at least a correspondence between a physical log and the information related thereto, which may have been stored, may be lost.

It has also been found that after storing and before debarking and/or peeling the log or a block cross-cut from the log, the log or the block may be analysed to determine secondary information. The secondary information needs not be as accurate as the primary information, and may thus be measured using less expensive equipment. Moreover, the secondary information can be used to locate and retrieve the primary information, which is more accurate and concerns the same log, from a database to which the primary information has been stored. In other words, the secondary information can be associated with the primary information stored in the database.

Thus, the method comprises accessing a database with the secondary information to receive the primary information from the database, and controlling a debarking apparatus and/or a lathe using the primary information of the log. In addition, the secondary information may be used for controlling the debarking apparatus and/or the lathe. The debarking apparatus and/or the lathe can be controlled before or during debarking and/or peeling of the log or the block. It has been noticed that the debarking apparatus can be controlled after debarking the log or the block.

The invention is disclosed in more specific terms in claim 1. Preferable embodiments are disclosed in the dependent claims. The description discloses also further embodiments.

### Brief description of the drawings

- Fig. 1: shows a method for manufacturing veneer,
- Fig. 2a: shows a part of a method for manufacturing veneer having a same width as a length of a log, and a use of the veneer,
- Fig. 2b: shows a part of a method for manufacturing veneer having a same width as a length of a block, which is cross-cut from the log, analysing the block, and a use of the veneer,
- Fig. 2c: shows a part of a method for manufacturing veneer having a same width as a length of a block, which is cross-cut from the log, analysing the log, and a use of the veneer,
- Fig. 3a: shows a log having knots,
- Fig. 3b: shows a core part, a surface part, and bark of a log,
- Fig. 3c: shows a log having curvedness,
- Fig. 4a: shows a log and a veneer obtainable from the log by peeling,
- Fig. 4b: shows a remainder of a log after peeling,
- Fig. 5a: shows a method for manufacturing veneer including imaging a log and analysing the log before debarking,
- Fig. 5b: shows a method for manufacturing veneer including imaging a log and analysing the log after debarking,
- Fig. 5c: shows a method for manufacturing veneer including imaging a second log and analysing the second log or a block cross-cut from the second log,
- Fig. 5d: shows a method for manufacturing veneer including cross-cutting a first log to form a first primary block and analysing the first primary block,
- Fig. 6: shows a method for manufacturing veneer including imaging a log, analysing the log, and imaging the veneer,
- Fig. 7a: shows in an end view peeling a log using a lathe and parts of the lathe,
- Fig. 7b: shows in top view peeling a log using a lathe and parts of the lathe,
- Fig. 8: shows a schematic of a debarking apparatus, and
- Fig. 9: shows imaging and disposing a low-quality log.

### Detailed description

Plywood or laminated veneer lumber (LVL) is manufactured by laying veneer sheets on top of each other, adhesive in between, and pressing them to form a panel, i.e. a plywood panel or a LVL panel. The veneer sheets are clipped from a large veneer web (hereinafter a veneer), and the veneer sheets and/or the veneer (i.e. veneer web) has been dried before pressing. Veneer is manufactured from logs. Most often the veneer is manufactured by peeling using a lathe.

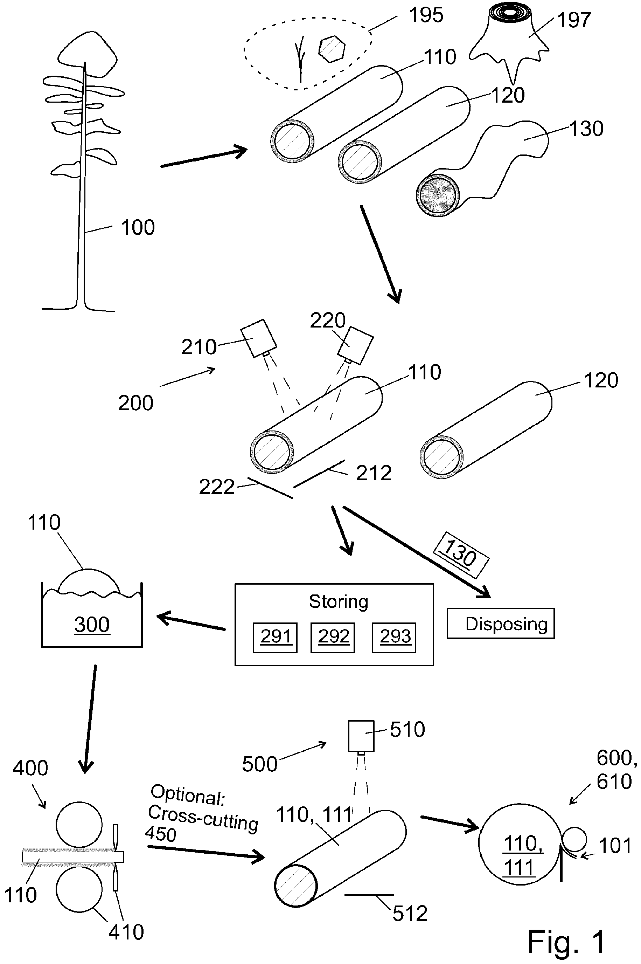

Referring to Fig. 1, manufacturing veneer 101 starts by felling trees 100 and dividing the resulting material to logs 110, 120, 130, stumps 197, and other wood material 195. The logs 110, 120, 130 are transported to a veneer manufacturing plant. Transportation may take place by any known means including transportation by a truck, by a ship, by a railway, and by rafting, including any combination thereof.

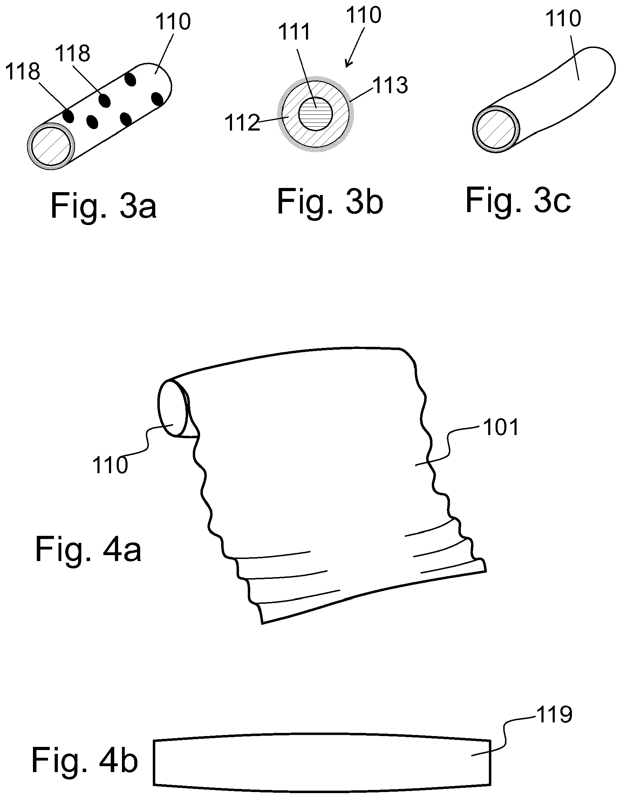

It has been found that the veneer manufacturing process can be optimized by classifying the logs 110, 120, 130 to different quality classes according to their properties related to quality. Such properties include amount of knots in the log, density profile of the log, presence of foreign objects (e.g. metal and/or rocks) in the log, presence of decay (e.g. rot, cracks) in the log, and shape (e.g. curvedness, thickness, circularity) of the log. Such properties may include also further aspects of the log, as detailed below. As an example, Fig. 3a shows knots 118 of a log 110. Fig. 3b shows a core 111 and a surface part 112 of a log 110, the core 111 comprising heartwood and the surface part 112 comprising sapwood. Typically the heartwood is denser than the sapwood. Typically the heartwood is harder than the sapwood. The density correlates positively with the hardness of the wood. However, hardness is also affected by temperature and moisture content. Temperature and moisture content of wood is affected by conditioning the logs or the blocks. Depending on multiple issues the heartwood/sapwood ratio varies, as well as the densities and/or hardnesses thereof. Moreover, at least before debarking, the log 110 may comprise bark 113. If the core 111 is rotten, it typically is less dense and softer. Further, Fig. 3c shows a log 110 that has some curvedness.

As for the shape of the log, while a minor curvedness is acceptable for veneer production, major curvedness, including multiple crookedness is unacceptable. Presence of a foreign object, such as a metal part or a rock in the log would destroy the knife of the lathe 610, whereby logs with foreign objects, such as nails, shots, bullets, and/or pieces of rock are unacceptable. Presence of decay may have the effect that the spindles of the lathe cannot reliably hold the log while peeling. The term decay herein refers to damaged material, including cracks and rot. As an example, if the core 111 is rotten, the log or the block cannot be reliably held by spindles of a lathe. Moreover, cracks may have the same effect.

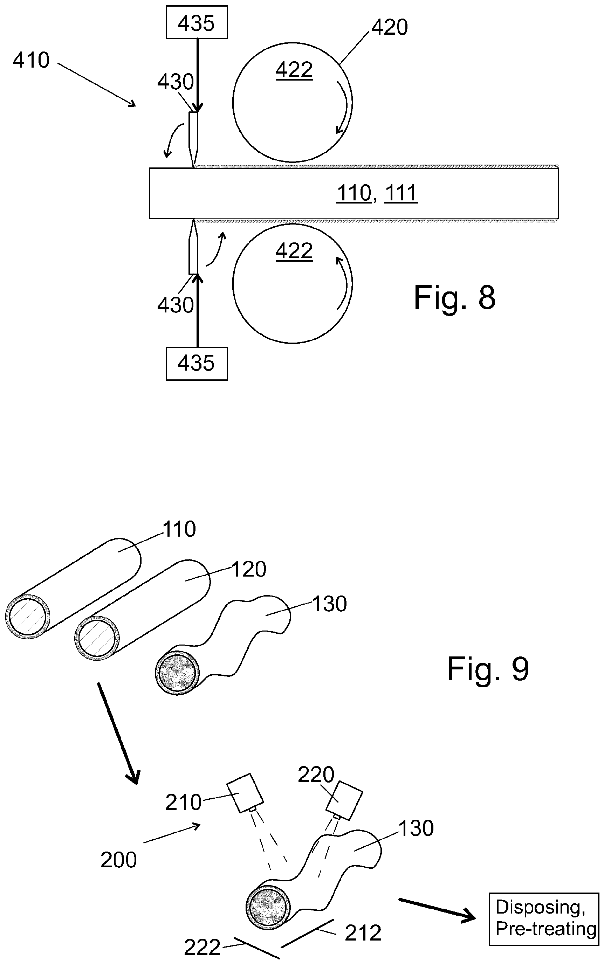

Fig. 9 shows imaging a third log 130, of which quality is too low for manufacturing veneer, whereby the third log 130 may be disposed before peeling. Typically such log is not even conditioned or debarked. If only a part of the log is unacceptable, it may be possible to saw off such part(s) from the log instead of disposing the whole log. Furthermore, instead of sawing off parts of the log before storing the log, the low-quality parts of the log may be marked so that the low-quality parts of the log may sawed off during cross-cutting 450.

Thus, instead of disposal, the log may be pre-treated before storing. The pretreatment may include sawing off a part of the log or marking a part of the log for removal. For example, if the log comprises a piece of metal (e.g. nail) near an end of the log, that part may be removed by sawing it off. This may be done before storing the log or during cross-cutting 450. As another alternative, if only an end of the log is cracked or highly curved, that part may be sawn off. It may be beneficial to pre-treat the logs, if needed, before storing. However, it may suffice to pre-treat the logs, if needed, later in the process, such as during cross-cutting 450, but before peeling.

Moreover, even if the information of knots in the first log, density profile of the first log, or the shape of the first log does not necessarily imply disposal or pretreatment of the log, these parameters may affect yield of high quality veneer obtainable from the log by peeling. Thus, it may be beneficial to store different types of logs in different storages. Moreover, these parameters may be usable for identifying the log in a later stage.

Information indicative of at least one of these properties is herein referred to as primary information. When it concerns a first log 110, it is referred to as first primary information 111. When the information concerns a second log 120, it is referred to as second primary information I21. When the information concerns a third log 130, it is referred to as third primary information.

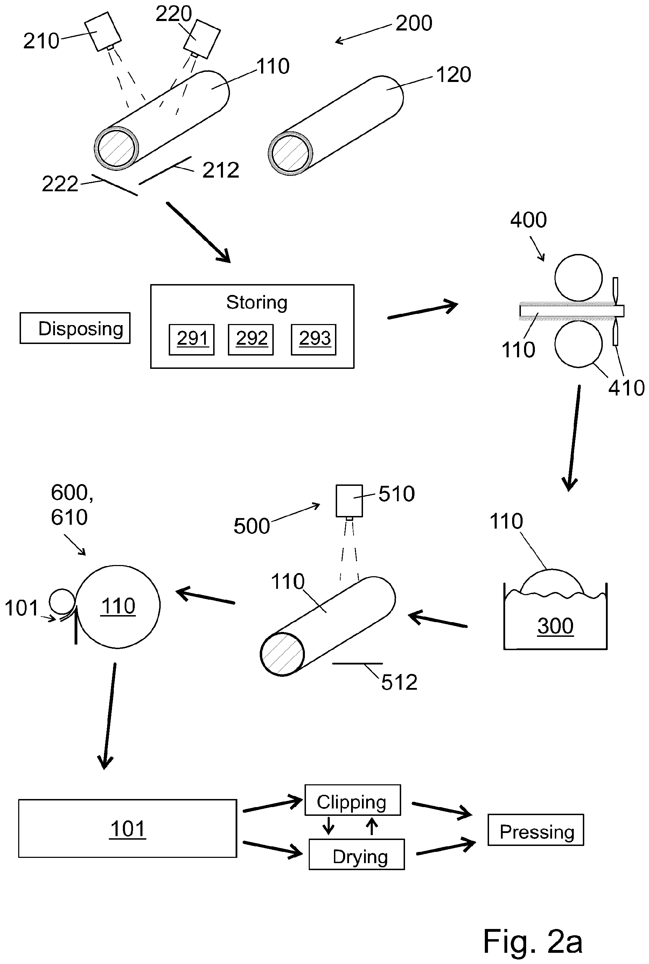

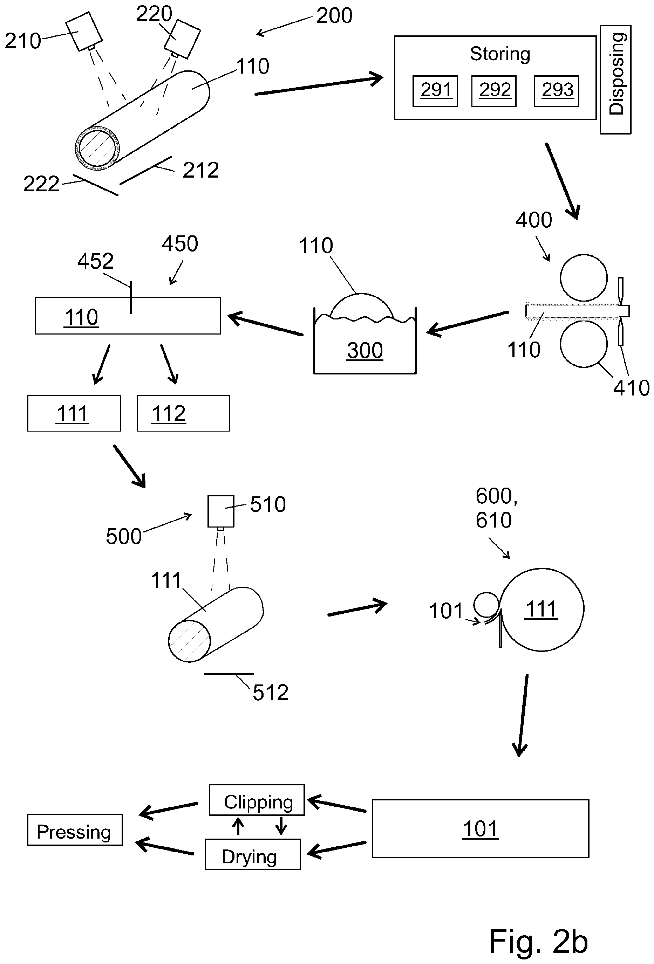

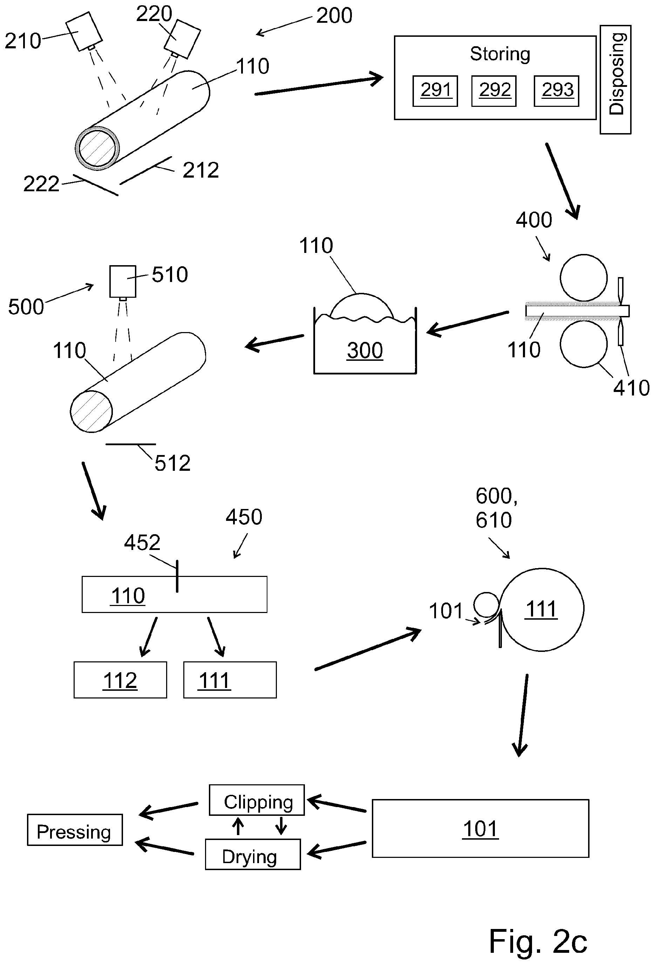

Thus, based on the primary information, the logs 110, 120, 130 can be classified as belonging to different quality classes. This classification and measurements related thereto may be performed at the veneer manufacturing plant. One of the quality classes may correspond to so low quality that the log is not to be used for veneer manufacturing at all (see Fig. 9) or that it needs to be pre-treated before storing or peeling (see above). As for the other logs, they may be stored in different storages 291, 292, 293 according to their quality class (see Figs. 1 and 2).

After storing, the logs or blocks cross-cut therefrom go through, in any order, debarking 400 and conditioning 300. Oftentimes a moisture content and a temperature of the logs/blocks is adjusted by soaking. In the alternative, a moisture content and a temperature of the logs/blocks is adjusted by steaming. Thus, the conditioning refers to a process wherein a temperature and/or a moisture content of the logs or blocks is conditioned. Conditioning 300 ensures that the log 110, 120 is suitably wet and warm (at least not frozen) for purposes of peeling 600. The moisture content and the temperature affects the plasticity of the wood, which is an important factor affecting peeling. The conditioning preferably comprises at least one of soaking and steaming. Moreover, preferably, the conditioning comprises soaking. Hereinafter, the process is described using soaking as an example of conditioning 300 in the figures.

In addition, depending on the length of the log, it may be cross-cut before peeling. In Fig. 1, the log 110 is conditioned 300 before debarking 400. Debarking makes the log 110, 120 suitable for peeling 600 so that the peeling 600 itself does not generate a lot of waste that might have drawbacks in the peeling 600.

If the logs are longer than the lathe (or lathes) can handle, the logs 110 may be cross-cut 450 before peeling. If the log is cross-cut, it may be cross-cut after storing, e.g. after conditioning or after debarking. If the log 110 is cross-cut, it is preferably cross-cut after conditioning and before peeling. Debarking may occur before or after the cross-cutting 450. If e.g. a first log 110 is cross-cut, at least two blocks, a first primary block 111 and a first secondary block 112, are obtained by cross-cutting 450 with a saw 452 (see Figs. 2b and 2c). As shown in the figures, cross-cutting 450 forms such a surface that has a normal that is substantially parallel to a direction of length of the log 110 that is cross-cut. An angle of up to 10 degrees may be left between the direction of length of the log and a normal of the surface formed by the cross-cutting.

Depending on whether the log is cross-cut and at what point of the process the log is cross cut, instead of conditioning the first log 110 the first primary block 111 may be conditioned. Moreover, instead of debarking the first log 110 the first primary block 111 may be debarked. For these reasons, either the log (110, 120, 130) or a block (111, 112, 121) cross-cut from the log, is peeled.

After debarking 400 and conditioning 300, the log or the block is peeled 600 using a lathe 610 to form the veneer 101 (i.e. veneer web).

Referring to Fig. 2a, the log 110 may be peeled, if a length of the log is suitable for a lathe 610 without cross-cutting. Referring to Figs. 2b and 2c, the log, e.g. a first log 110, may be cross-cut by a saw 452 to form a first primary block 111 and a first secondary block 112. Thereafter, the first primary block 111 is peeled. This is depicted also in Fig. 1 by the optional cross-cutting. Also the first secondary block 112 may be peeled. However, the first secondary block 112 may be disposed, e.g. if a quality thereof is too low and/or a length thereof is not suitable for peeling. A log 110 may be cross-cut to form three blocks or four blocks, or any other suitable number of blocks.

Different logs 110, 120 or blocks originating from different logs may be peeled using different lathes. E.g. all logs or blocks obtainable from logs from a first storage 291 may be peeled with a first lathe 610 and all logs or blocks obtainable from logs from a second storage 292 may be peeled with a second lathe. In addition or alternatively, logs from different storages 291, 292, 293, or different blocks obtainable from such logs, may be conditioned 300 for different periods of times and/or at different temperatures.

Figs. 2a to 2c show embodiments of a method for manufacturing veneer 101 including, as an example, a use of the veneer. The steps before storing (or disposing) are the same as in Fig. 1.

Referring to Fig. 2a, after peeling 600, the veneer 101 is clipped to veneer sheets and dried. Drying may be done before clipping (i.e. the whole veneer is dried) or thereafter (i.e. the veneer sheets are dried). As detailed above, thereafter the veneer sheets are laid up to form a stack, provided with adhesive, and pressed together to form the plywood panel or the LVL boards, which may be sawn according to needs (e.g. for finalizing the edges and/or to form smaller pieces). Therefore, an embodiment comprises clipping the veneer 101 to obtain veneer sheets and thereafter drying the veneer sheets. An embodiment comprises drying the veneer 101 and thereafter clipping the veneer 101 to obtain veneer sheets. The veneer 101 can be used in a similar manner in the context of Figs. 1, 5a, 5b, 5c, 5d, and 6, too.

It has been found that the yield of high quality veneer 101 can be improved in two ways: (1) by optimising the peeling 600 log-wise so that an amount of discarded veneer material can be optimized (e.g. minimized), and conversely the yield optimized (e.g. maximized and concerning particularly the high quality veneer); and/or (2) by optimising the debarking 400 so that only the needed amount of bark 113 is removed from the log 110 by the debarking 400 with the best debarking settings. Herein the term best debarking settings refer to debarking parameters such that substantially all the bark is removed, but, at the same time, the quality of the sapwood of the log (or block) is not compromised with. It has been found that both these optimizations may be based on the primary information 111 of the log 110.

Hereinafter, the method is more specifically described for manufacturing veneer 101 from a first log 110. Naturally, veneer (i.e. another veneer web) may be manufactured in a similar manner from a second log 120. Furthermore, referring to Fig. 9, a quality of a third log 130 may be so low that it is disposed. As detailed above, a low-quality log may be pre-treated to improve its quality.

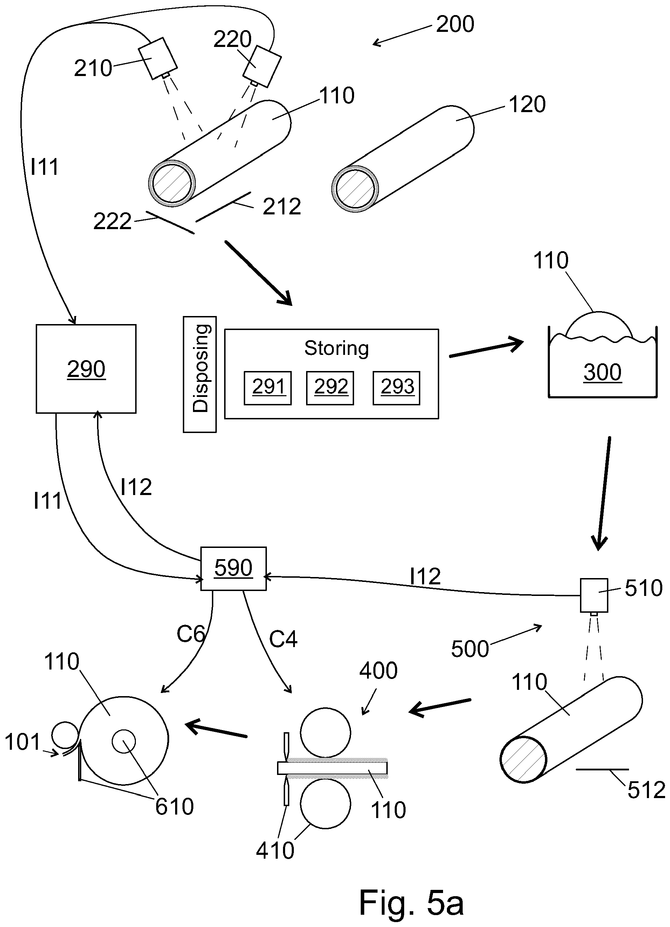

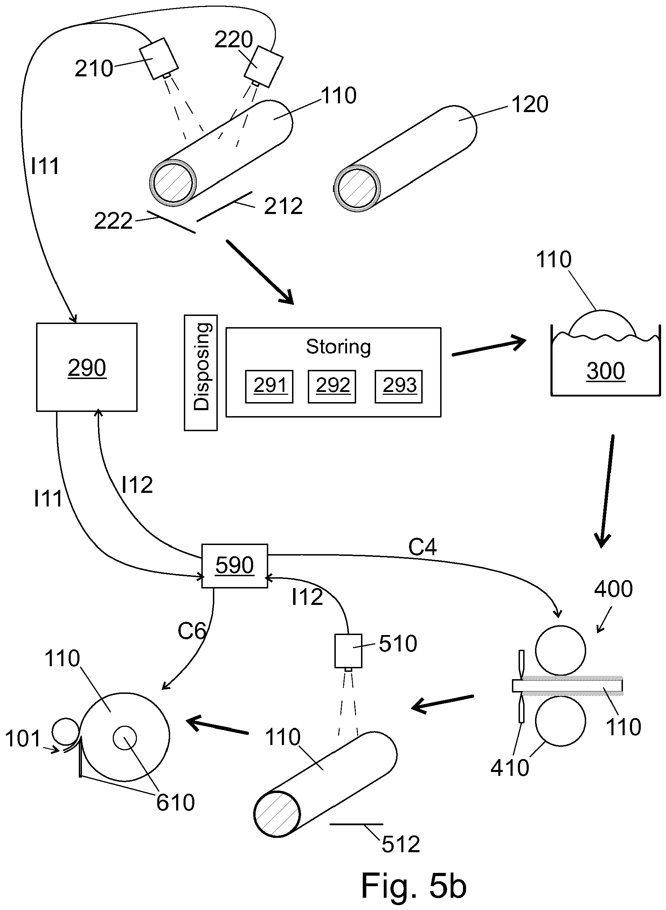

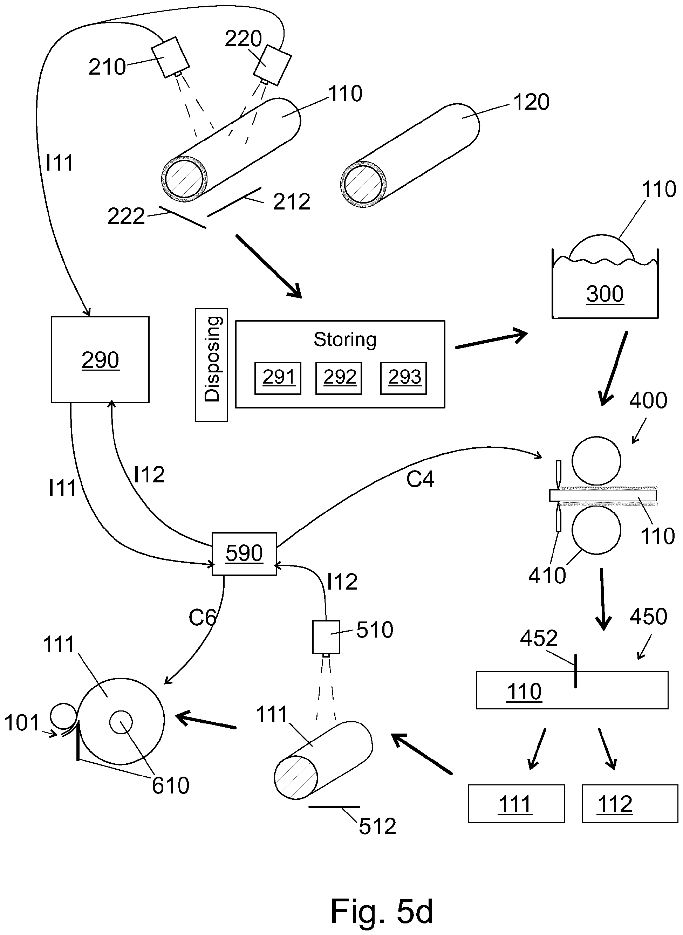

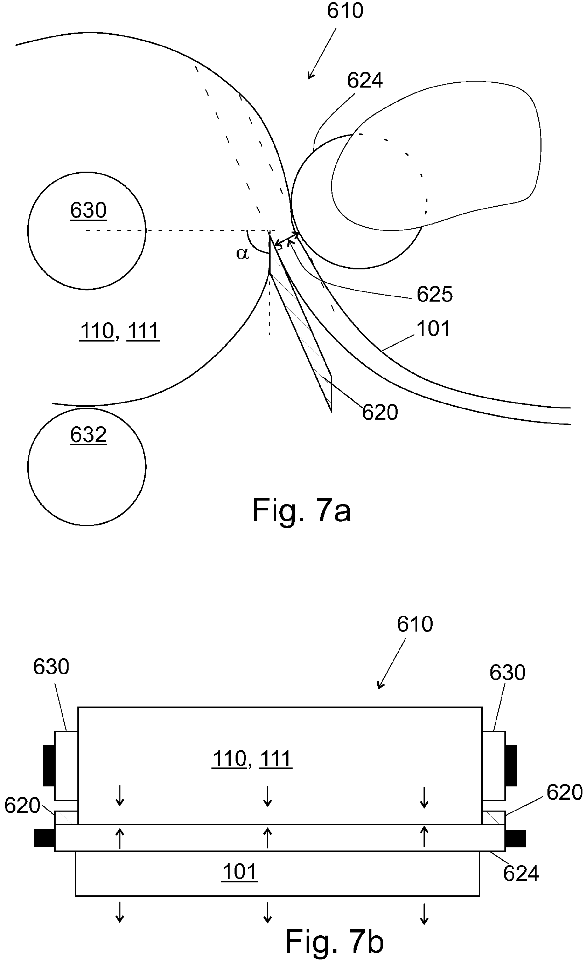

Referring to Figs. 5a, 5b, and 5d the method for manufacturing the veneer 101 comprises, in any order, conditioning (e.g. soaking) 300 the first log 110 or the first primary block 111 and debarking 400 the first log 110 or the first primary block 111 using a debarking apparatus 410. After conditioning 300 and debarking 400, the method comprises peeling 600 the first log 110 or the first primary block 111 using a lathe 610 to form the veneer 101. In Fig. 5a, only a spindle and a knife of the lathe 610 are shown. For some other parts of a typical lathe, reference is made to Figs. 7a and 7b. However, a lathe 610 needs not comprise a spindle, when it comprises rotators configured to rotate the first log 110 or the block 111 otherwise.

Referring to Fig. 5a, the method comprises before the conditioning 300, the debarking 400, and the peeling 600 of the first log 110 or the first primary block 111, imaging 200 the first log 110 a first time to determine first primary information 111 of the first log 110, the first primary information 111 being indicative of at least one of the following: (i) information of knots 118 in the first log 110 (see Fig. 3a), (ii) density profile of the first log 110 (see Fig. 3b), and (iii) shape of the first log 110 (see Fig. 3c). Preferably the method comprises imaging a surface of the first log 110 and an inner part of the first log 110, the inner part of the first log 110 being arranged beneath the surface of the first log 110. The inner part comprises e.g. the core 111 (see Fig. 3b) of the first log 110. Thus, the first primary information 111 of the first log 110 is determined. The inner part of the first log 110 can be imaged using e.g. X-ray. The surface can be imaged using one or more laser scanners and/or a camera. More specific examples will follow.

Concerning the information of knots, the information of knots may be indicative of dimensions, densities, locations, directions, and types of the knots 118 of the of the first log 110, as well as a number density of the knots of the first log. The number density refers to a number of the knots in a volume or area of the log and may be spatially distributed. As for the type of the knots, the knots may be e.g. dry, fresh, rotten, or dead. In the context of second primary information 121, the information of knots may be indicative of dimensions, densities locations, directions, and types of the knots 118 of the of second log 120; or a number density of the knots of the second log 120. Dimensions of a knot may include a diameter and a depth or a height from a surface of the log.

Imaging at least the inner part of the first log 110 has the benefit that the density profile can be determined. The density profile can be used for controlling the lathe 610, because yield can be increased by peeling hard wood somewhat differently than soft wood; and the density is indicative of hardness. Moreover, the density profile may be indicative of proportions of sapwood and heartwood, and qualities of sapwood and heartwood may vary; which may be taken into account when peeling. The density profile can be used for controlling the debarking apparatus 410, because the density profile is indicative of thickness of bark 113 and softness of a surface part 112 of the log.

In an embodiment, the first primary information 111 is also indicative of at least one of a length of the first log 110, a diameter of the first log 110, a diameter of the first log 110 as a function of position from an end of the first log 110, a shape of the first log 110, a conicity of the first log 110, an ellipticity of the first log 110, a multiple crookedness of the first log 110, location(s) of low-quality wood in the of the first log 110, presence or amount of rot in the of the first log 110, presence of cracks in the first log 110, proportion of sapwood and core wood of the first log 110, a peripheral area of the first log 110, a thickness or amount of bark of the first log 110, thicknesses of tree rings in the first log 110, and a volume of the first log 110. Also these parameters affect the quality of the first log 110. The term quality, in connection with a log (e.g. 110), may refer to an amount of high quality veneer 101 that is obtainable from the log (e.g. 110).

As for the quality of the log, in terms of classifying the log it is beneficial that the first primary information 111 is also indicative of at least one of: (iv) absence of foreign objects from the first log 110, and (v) absence of decay from the first log 110. Presence of foreign object, such as a metal piece or a rock, may indicate that the log cannot be peeled at all. In a similar manner, presence of decay may indicate that the log is not valid for regular peeling. Correspondingly, an absence thereof may indicate the ability of the log for being peeled.

In an embodiment, the first primary information 111 is indicative of all of (i) information of knots 118 in the first log 110, (ii) density profile of the first log 110, (iii) shape of the first log 110, (iv) absence of foreign objects from the first log 110, and (v) absence of decay from the first log 110.

Referring to Fig. 5a, the method comprises storing the first primary information 111 in a first database 290. As detailed above, in a preferable embodiment, the first primary information I11 is used for classifying the first log 110, and the information I11 is therefore needed in that embodiment before storing the first log 110. Thus the step of imaging 200 is before the steps of conditioning 300 and debarking. Moreover, if the first log 110 is stored, the step of imaging 200 is before storing. Since the first primary information I11 is used also later, it is stored in the first database 290 for later use.

Preferably, when the first primary information I11 of the first log 110 is stored to the first database 290, the first primary information I11 is stored in association with an identifier (e.g. identity number) for the first log 110. Thus, an embodiment comprises forming an identifier for the first log 110 and storing the identifier in association with the first primary information I11 to the first database 290. This is particularly feasible, when an identity of the first log 110 can be obtained by reading an information carrier or information marker in the step of analysing 500 the first log 110 or the first primary block 111 a second time to determine first secondary information I12.

The method comprises, after imaging 200 the first log 110 the first time, analysing 500 the first log 110 or the first primary block 111 a second time to determine first secondary information I12. Thus, the first secondary information I12 comprises information of the first log 110 or the first primary block 111. In embodiment, the first log 110 or the first primary block 111 is analysed 500 the second time after conditioning 300 the first log 110 or the first primary block 111 and before peeling 600 the first log 110 or the first primary block 111, to determine the first secondary information I12. The first secondary information I12 comprises such information that allows for finding, from the first database 290, the first primary information I11 concerning the first log 110. Moreover, because the first primary information I11 is used for the process control, the method comprises accessing the first database 290 with the first secondary information I12 to receive the first primary information I11 from the first database 290. Thus, the method comprises receiving the first primary information I11 from the first database 290. In a preferred embodiment, the analysing 500 involves imaging and imaging can be seen as a form of analysing, whereby for clarity, the step of imaging 200 is done a first time and the step of analysing 500 is done a second time.

As readable from above, the first secondary information I12 is used mainly to retrieve correct first primary information I11 from the first database 290. The term "correct" herein means that that after having analysed 500 the first log 110 or the block 111, the primary information I11 concerning particularly the first log 110 is received from the first database 290. Naturally the first database 290 may comprise primary information concerning multiple other logs, too (e.g. the logs 120, 130).

In a veneer manufacturing plant, several logs or blocks are processed subsequently. Therefore, after determining the first secondary information I12, the first primary information should be received from the first database 290 reasonably fast, because otherwise it might happen that when the first primary information I11 is used (as detailed above and below), the log or block that would be debarked or peeled would not the one, from which the first primary information I11 has been obtained. If the delay was long, it might happen, that a subsequent log/block would have already entered the debarking apparatus or lathe because of delays in the processing. Thus, receiving the first primary information I11 should only take at most as long as it takes time from the log/block to move from the step of analysing 500 to the step where the information is used (i.e. debarking, if the information I11 is used for debarking; or peeling, if the information I11 is used in peeling). How much there is time depends on throughput of the veneer manufacturing plant; the higher throughput, the less time. A typical time frame (i.e. maximal delay) for retrieving the first primary information with a typical throughput is from 3 s to 30 s, depending on what is considered as typical throughput.

For these reasons, an embodiment comprises analysing the first log 110 or the first primary block 111 at a first instance of time, accessing the first database 290 with the first secondary information I12 to receive the first primary information I11 from the first database, and receiving the first primary information I11 from the first database 290 at a second instance of time, wherein a difference between the second instance of time and the first instance of time is at most 30 seconds.

In order to reduce the difference between the second instance of time and the first instance of time, an embodiment comprises forming statistical data of the first log 110. More specifically, in an embodiment, the step of imaging 200 the first log a first time to determine first primary information I11 comprises imaging the first log 110 a first time to obtain an image and forming statistical data of the first log 110 from the image such that the first primary information I11 comprises the statistical data of the first log 110.

Referring to Figs. 2a, 5a, and 5b, in an embodiment, a length of the first log 110 is suitable for the lathe 610 as such. Thus, cross-cutting is not needed. Therefore, the analysing 500 involves analysing the first log 110. When the first database is accessed with the first secondary information I12, the first primary information I11 concerning the first log 110 is received from the first database 290, and the first primary information I11 is used to control the lathe or the debarking apparatus.

Referring to Figs. 2b, 2c, and 5d, in an embodiment, the first log 110 is so long that at least a first primary block 111 and a first secondary block 112 needs to be cross-cut from the first log 110 so that the first primary block 111 fits the lathe 610. Correspondingly, the first log 110 would not fit the lathe 610. In this case there are at least two possibilities for the order different steps of the process.

Referring to Figs. 2b and 5d, in an embodiment, the first log 110 is cross-cut to the blocks 111, 112 before analysing 500. Therefore, the first primary block 111 is analysed to receive the first secondary information I12. When the first database 290 is accessed with the first secondary information I12, the first primary information I11 concerning the first log 110 is received from the first database. Moreover, by comparing the first secondary information I12 with the first primary information I11, the part of the first primary information I11 of the first log 110 that describes the properties of the first primary block 111 is selected. Thereafter, the part of the first primary information I11 that describes the properties of the first primary block 111 is used to control the lathe 610 and/or the debarking apparatus 410. Moreover, the first primary information I11 can be used for the first secondary block 112 in a similar manner. Thus, the part of the first primary information I11 that describes the properties of the first secondary block 112 can used to control the lathe 610 and/or the debarking apparatus 410 while or before peeling/debarking the first secondary block 112. However, the first secondary block 112 need not be peeled. For example, if the first primary information I11 indicates that a quality of the first secondary block 112 is too low, the first secondary block 112 may be disposed at this stage. Moreover, if a length of the first secondary block 112 is unsuitable for a lathe of the veneer manufacturing plant, the first secondary block 112 may be used otherwise or disposed.

Referring to Fig. 2c, in an embodiment, the whole first log 110 is analysed to determine the first secondary information I12 before the cross-cutting 450 of the first log 110 to the blocks 111, 112, of which at least the first primary block 111 is peeled to form veneer. The first primary information I11 can be received from the first database 290 is detailed above. Thereafter, it is mainly a matter of peeling the block(s) 111, 112 in such an order that the part of the first primary information I11 that describes the properties of the first primary block 111 is used to control the lathe 610 and/or the debarking apparatus 410 e.g. when peeling/debarking the first primary block 111. If the first secondary block 112 is peeled, that the part of the first primary information 111 that describes the properties of the first secondary block 112 may be used to control the lathe 610 and/or the debarking apparatus 410 e.g. when peeling/debarking the first secondary block 112.

For associating the first secondary information 112 with the first primary information 111 there are at least two mutually non-exclusive options.

As a first option, an information carrier, or multiple information carriers, may be attached to the first log 110, the information carrier(s) comprising an identity of the first log 110 or the block(s) 111, 112. In case the first log 100 is cross-cut e.g. to form the blocks 111, 112, one information carrier corresponding to each block 111, 112 may be attached to the first log 110. For example, if two blocks 111, 112 are cross-cut from the log 110, one information carrier may be attached or printed to each end of the first log 110. The information of these information carriers, e.g. identities of the information carrier, may be stored in the first database 290 in connection with an identity of the first log 110. As an alternative to information carrier(s), an information marking, or multiple information markings, may be printed to the first log 110. The printing may be done e.g. with a laser or by using water-proof ink. The printing may be done directly on wood or on a label, whereby the label may serve as the information carrier. E.g. an information marking can be laser-printed on both ends of a log.

In this embodiment, the step of analysing 500 the first log 110 or the block 111 comprises reading the information carrier or the information marking. Referring to Fig. 2c, the information carrier or information marking may be read before cross-cutting 450 and referring to Fig. 2b, the information carrier or information marking may be read after cross cutting. Referring to Fig. 2a, an information carrier or information marking may be used even if the log 110 is not cross-cut. As an identity of the first log 110 or the block 111 may be read from the information carrier or information marking, the identity can be used to retrieve the first primary information I11 form the first database 290. Correspondingly, in the embodiment, the information on the information carrier or information marking is used for associating the first primary information I11 of the first database 290 with the first secondary information I12 to receive the first primary information I11 from the first database 290. Examples of an information carrier include an RFID tag and a label comprising a one-dimensional barcode, or a two-dimensional barcode (e.g. a QR-code). Examples of information markings include a one-dimensional barcode and a two-dimensional barcode (e.g. a QR-code). The one or two-dimensional barcode may be printed directly to the first log 110 or the one or two-dimensional barcode may be printed on a label (e.g. an adhesive label) that is fixed (e.g. attached) to the first log 110. As indicated above, the first primary information I11 may be stored to the first database 290 in association with an identifier of the log 110 and/or with identifiers of the blocks 111, 112. Thus, the information carrier(s) of the first log 110 may comprise information indicative of the identifier(s) of the first log 110 and/or the first primary block 111 and the first secondary block 112 manufacturable from the first log 110 by cross-cutting.

As a second option, a separate information carrier or information marking is not needed. In such a case, the step of analysing 500 the first log 110 or the first primary block 111 the second time comprises imaging the first log 110 or the first primary block 111 to receive a second image of the first log 110 or the first primary block 111. Typically an image of the first log 110 or the first primary block 111, at least if sufficiently accurate and/or if combined with other data (including X-ray images and/or shape information from scanners) is sufficiently accurate for associating the first secondary information I12 correctly to the first primary information I11. The embodiment comprises using the second image of the first log 110 or the first primary block 111 for associating the first primary information I11 of the first database 290 with the first secondary information I12 to receive the first primary information I11 from the first database 290. The second image of the first log 110 or the first primary block 111 needs not be as accurate as the image obtained in the step of imaging 200 the first log 110, as detailed below.

As for obtaining sufficiently accurate first secondary information 112 from the first log 110 or the first primary block 111, at least when a separate information carrier or information marking is not used, preferably the step of analysing 500 the first log 110 the second time comprises
- imaging the first log 110 or the first primary block 111 to obtain the second image of the first log, the second image being indicative of information of knots 118 in the first log 110 or the first primary block 111, and
- scanning the first log 110 or the first primary block 111 to obtain information indicative of shape of the first log.

More preferably, the step of analysing 500 the first log 110 or the first primary block 111 the second time in addition comprises imaging the first log 110 from one direction with X-ray to obtain an image of an inner part of the first log 110 or the first primary block 111.

The measurements for the first secondary information I12 may be similar to those for obtaining the first primary information I11. However, preferably, in the step of analysing 500 the first log 110 or the first primary block 111 a second time to determine first secondary information I12 only simpler measurements are made than in the step of imaging 200 the first log 110 a first time to determine first primary information I11 of the first log 110. This allows for using simpler devices for the measurements of the first secondary information I12, which shows in investment costs, and also operational costs, because simpler devices are typically more robust, more reliable, and require less maintenance. Thus, in an embodiment, the step of analysing 500 comprises using fewer sensor components than the step of imaging 200.

For these reasons, the first primary information I11 is only measured once and stored in the first database 290, and the first secondary information I12 is used for purposes of retrieving the first primary information I11 from the first database 290. In an embodiment, the first primary information 111 is more accurate than the first secondary information I12. The first secondary information I12 may comprise such information that is a subset of the first primary information I11. E.g. the first secondary information I12 may contain, in addition to some other data (e.g. a scanned shape), an X-ray image of the log 110 or the block 111, and the first primary information I11 may contain a three-dimensional image of the log 110. In this way the part of 112 that is a subset of some primary information (e.g. first primary information 111, second primary information 121 concerning a second log 120, and third primary information 131 concerning a third log 130) can be easily compared with that part of each one of 131, 121, 111 to determine which one of these pieces primary information corresponds to the first secondary information 112; i.e. which one of the primary information I11, I21, I31 correspond to the same first log 110 as the first secondary information 112. Moreover, at the same time it can be determined, which part of the primary information corresponds to the secondary information of a block, if the log is cross-cut to form blocks and only the blocks are analysed to form the first secondary information I12.

As an example, *a priori,* it is not known that certain I11 corresponds to I12, because the different logs get mingled when stored. However, after comparison, one can be determine that the part of I12 that is a subset of 111 is identical or substantially similar to that subset of 111. Thus, a *posteriori,* one can determine that I11 corresponds to I12, both concerning the same first log 110. As readable from above, the secondary information should be sufficiently distinctive, i.e. a *posteriori,* only one of the pieces of primary information I11, 121, 131 (which are obtained from a first log 110, a second log 120, and a third log 130, respectively) corresponds to I12.

Moreover, the image as such obtained in the step of analysing 500 need not be compared with the image as such obtained in the step of imaging 200. It has been found that associating the first secondary information 112 with the first primary information 111 can be made much faster, when statistical data is formed of the images, and the statistical data are compared for associating the pieces of information 112, 111 with each other. Such statistical information may comprise e.g. a number and locations of knots, a diameter and a roundness of the log, and curvedness of the log.

As for the process control, the method comprises [A] controlling the debarking apparatus 410 using the first primary information I11 of the first log 110 and/or [B] controlling the lathe 610 using the first primary information I11 of the first log 110. Preferably the method comprises [A] controlling the debarking apparatus 410 using the first primary information I11 of the first log 110 and/or [B] before or while peeling 600 the first log 110 or the first primary block 111, controlling the lathe 610 using the first primary information I11 of the first log 110. More preferably the method comprises before or while peeling 600 the first log 110 or the first primary block 111, controlling the lathe 610 using the first primary information I11 of the first log 110.

In addition to the first primary information I11, the first secondary information I12 may be used for controlling the debarking apparatus and/or the lathe. This may be beneficial, because the logs or the blocks may be damaged in the process. Therefore, the first secondary information I12 may be, in some sense, more accurate than the first primary information I11.

Referring to Figs. 5a and 5b, the control may be performed by a processing unit 590. In the alternative, an operator (i.e. a human operator) may receive the first primary information I11 from the first database 290 and control the debarking apparatus 410 and/or the lathe 610. If only a block 111, 112 is peeled, the operator may receive all the first primary information I11 or only such part of the first primary information I11 that corresponds to the block 111, 112 that is peeled or debarked. However, preferably a control unit 590 is used at least to retrieve the first primary information I11 from the first database 290, e.g. for the operator to use the data in the peeling 600 and/or debarking 400. In Figs. 5a and 5b, the reference C6 indicates a control signal sent to the lathe 610 and the reference C4 indicates a control signal sent to the debarking apparatus 410.

Preferably the logs (110, 120, 130) are classified to different classes of log. The different classes of log may be indicative of e.g. a quality of the logs of that class. The quality may depend e.g. on the parameters of a log as discussed above in the context of the first log 110. The classes of logs may include at least two classes: one for logs that are used, and another for logs that are disposed or pre-treated (e.g. sawn) before use. However, preferably more classes of logs are used, because this enables better optimization of use of the logs. Thus, an embodiment comprises, after imaging 200 the first log the first time and before the conditioning 300, the debarking 400, and the peeling 600 of the first log, using the first primary information I11 of the first log 110 to classify the first log 110 such that the first log 110 belongs to a first class of logs and does not belong to a second class of logs. The embodiment further comprises transferring the first log 110 to a first storage 291 that comprises only logs that belong to the first class of logs. Thereafter, the first log 110 is stored for some time in the first storage 291 before conditioning 300 or debarking 400, whichever is made next after storing.

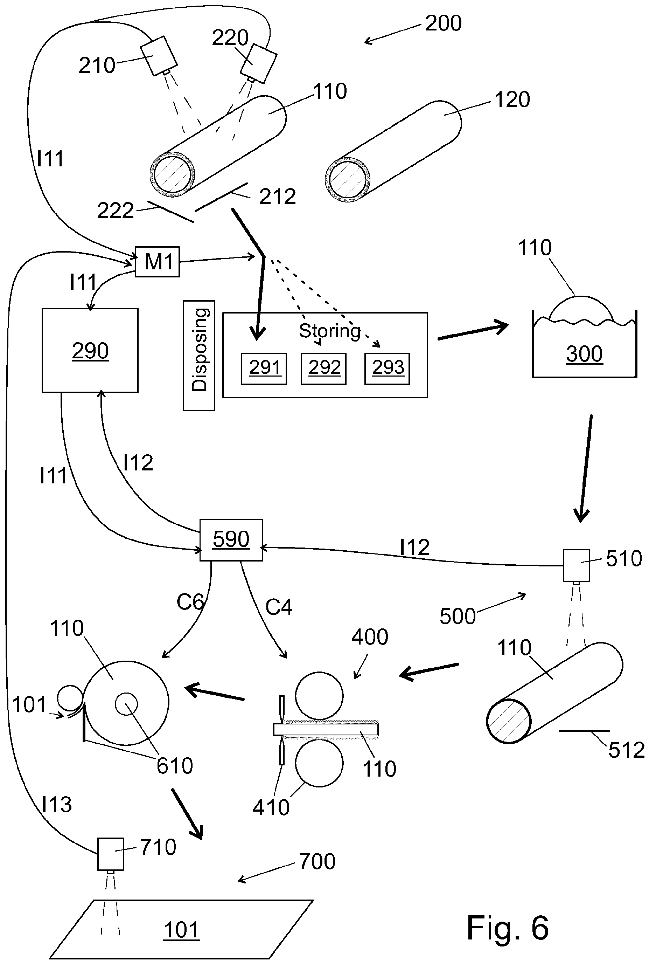

Preferably, the classification of the logs is automated. Referring to Fig. 6, an embodiment comprises using a first model M1 and the first primary information I11 of the first log 110 to classify the first log 110 such that the first log 110 belongs to a first class of logs and does not belong to a second class of logs. As indicated in Fig. 6 by a thick arrow, the first log 110 is then transferred to the first storage 291. Naturally, if the quality of the first log, as determined by an operator or the model M1 would be different, the first log 110 could be transferred to a second storage 292 or a third storage 293 as indicated by dash lines. Moreover, the first log could be pre-treated, such as sawn or marked for removal, to make it usable by removal of low quality wood. Moreover, the first log could be completely disposed, but then no veneer 101 would not be made from the first log 110.

The model M1 may be a numerical model, statistical model, or an artificial intelligence model. The model M1 may be created once some data is available. Moreover, after using the model M1, the model M1 may be updated (i.e. improved) to make better classification. The updating may be made by teaching the model. In the alternative, the updating can be done by re-creating the model with more data. The model M1 may be a data driven artificial intelligence model. This has the benefit that a quality of the veneer 101 can be used to teach the model M1 in use to improve the accuracy of classification. Teaching is a way of updating the model.

Referring to Fig. 6, for these reasons, an embodiment comprises after peeling 600 the first log 110 or the first primary block 111, imaging 700 the veneer 101 to obtain first tertiary information I13. An embodiment comprises creating the first model M1 using the first tertiary information I13 and the first primary information I11, the first model M1 being configured to classify a second log 120 based on second primary information I21 such that the second log 120 belongs to a first class of logs and does not belong to a second class of logs.

However, if the first model M1 has already been created, the first tertiary information I13 can be used to update the model M1. Therefore, an embodiment comprises updating (such as teaching) the first model M1 using the first tertiary information I13 and the first primary information I11. In this way, the fist model M1 is updated to recognize, from the first primary information I11 concerning the first log 110 an amount of high-quality veneer that is obtainable from such a log 110. Thus, the first tertiary information I13 is indicative of an amount of veneer 101 manufacturable from the first log 110, and in this way indicative of a quality of the first log 110. In this way, the quality-based storing of the logs can be improved during operation.

As indicated above, one reason for using the first database 290 is the storing of the first log 110. In particular, the storing of the first log 110 may last so long that before the first log 110 or the first primary block 111 is peeled, a second log 120 is received, imaged (in a similar way as the first log 110), classified, and stored (or pre-treated or disposed). Thus, when the first log 110 or the block 111 is debarked or peeled, the latest measurement of the primary information does not necessarily concern the first log 110. This emphasizes the need for the first database 290.

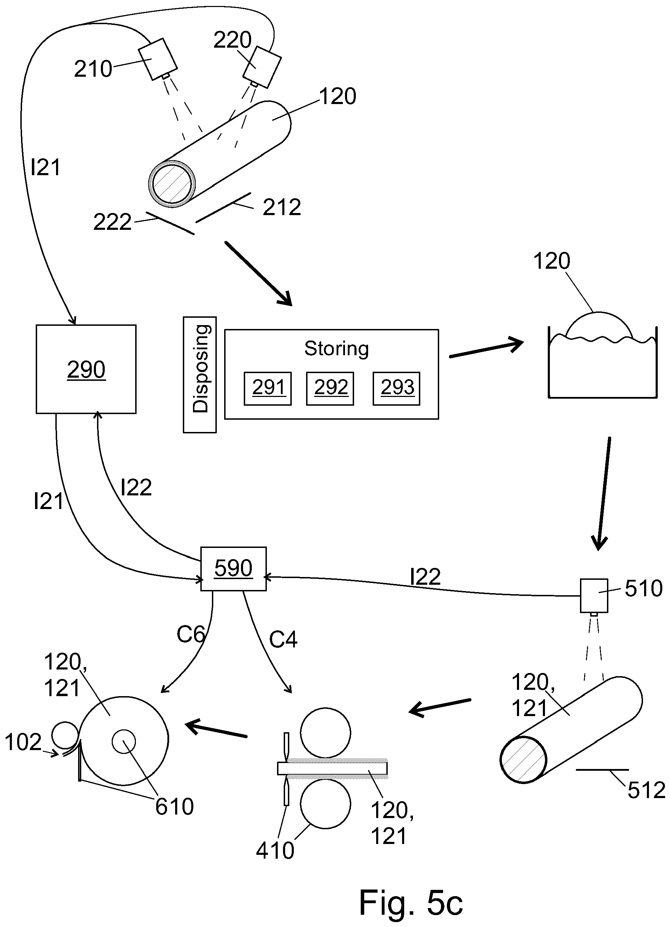

For these reasons, and with reference to Fig. 5c, an embodiment comprises after imaging 200 the first log 110 the first time and before analysing 500 the first log 110 or the first primary block 111 the second time, imaging a second log 120 a first time log to determine second primary information I21 of the second log, the second primary information I21 being indicative of at least one of the following: (i) information of knots in the second log 120, (ii) density profile of the second log 120, and (iii) shape of the second log 120. Then the second primary information I21 is stored in the first database 290. Preferably, the second primary information I21 is stored in the first database 290 before accessing the first database 290 with the first secondary information I12, which concerns the first log 110 or the first primary block 111, to receive the first primary information I11, which concerns the first log 110, from the first database 290.

For the same reasons as discussed in connection with the first log, preferably, the second primary information I21 is also indicative of at least one of the following: (iv) absence of foreign objects from the second log 120, and (v) absence of decay from the second log 120.

Moreover, the embodiment preferably comprises after the conditioning the second log 120 (or a block receivable from the second log 120 by cross-cutting) and before peeling the second log 120 (or a block receivable from the second log 120 by cross-cutting), analysing the second log 120 (or a block receivable from the second log 120 by cross-cutting) a second time to determine second secondary information I22; and accessing the first database 290 with the second secondary information I22 to receive the second primary information 121 from the database. Finally, the embodiment comprises
- controlling the debarking apparatus that debarks the second log or the block using the second primary information 121 of the second log 120 and/or
- controlling the lathe that peels the second log or the block using the second primary information I21 of the second log 120.

Preferably, the embodiment comprises
- controlling the debarking apparatus that debarks the second log 120 or a block 121 receivable from the second log 120 by cross-cutting using the second primary information I21 of the second log 120 and/or
- before or while peeling the second log 120 or a block 121 receivable from the second log 120 by cross-cutting, controlling the lathe that peels the second log or the block using the second primary information I21 of the second log 120.

More preferably, the embodiment comprises
- before or while peeling the second log 120 or a block 121 receivable from the second log 120 by cross-cutting, controlling the lathe that peels the second log or the block 121 using the second primary information I21 of the second log 120.

The debarking apparatus that debarks the second log 120 or the block 121 receivable therefrom may be the same debarking apparatus 410 that is configured to debark the first log 110 or the first primary block 111; or it may be another debarking apparatus. The lathe that peels the second log 120 or the block 121 receivable therefrom may be the same lathe 610 that is configured to peel the first log 110 or the first primary block 111; or it may be another lathe. In Fig. 5c, the second log 120 or the block 121 is peeled in the same lathe 610 as the first log 110 in Fig. 5a. Moreover, in Fig. 5c, the second log 120 or block 121 is debarked in the same debarking apparatus 410 as the first log 110 in Fig. 5a. The veneer 102 of Fig. 5c comes from the second log 120 or block 121 and can be used in the same way as the veneer 101 of Fig. 5a. Both can be considered to be veneer (material in the sense of a non-countable word) or a veneer web (i.e. veneer), in the sense of a countable word.

Preferably also the second log 120 is classified in a similar manner as the first log. Thus, an embodiment comprises after imaging the second log 120 the first time and before the conditioning, the debarking, and the peeling of the second log 120 or block 121, using the second primary information I21 of the second log 120 to classify the second log 120 such that the second log 120 belongs to a second class of logs and does not belong to a first class of logs, and transferring the second log 120 to a second storage 292 that comprises only logs that belong to the second class of logs.

Naturally, the second log 120 could have such a class that it would belong to the first class of logs and correspondingly be transferred to the first storage 291. The classification may be made by an operator or by the first model M1. Preferably, the classification is automated.

Thus, preferably, the embodiment comprises after imaging the second log 120 the first time and before the conditioning, the debarking, and the peeling of the second log, using the first model M1 and the second primary information I21 of the second log 120 to classify the second log such that the second log belongs to a second class of logs and does not belong to a first class of logs.

Logs taken from different storages 291, 292, 293 can be sent to different lathes and/or conditioned for different times and/or conditioned at different temperatures. However, logs taken from different storages 291, 292, 293 can be peeled using the same lathe and/or debarked using the same debarking apparatus, in particular, since these devices are controlled using the primary information (I11, I21) to have proper peeling and/or debarking parameters (e.g. peeling speed). Naturally, all logs taken from only one storage, e.g. 291, 292, or 293, can be peeled using the same lathe and/or debarked using the same debarking apparatus.

Thus, an embodiment comprises transferring at least a part of the first log 110 (such as a block 111 thereof) from the first storage 291 to the lathe 610 for peeling the first log 110 or the first primary block 111 and transferring at least a part of the second log 120 (such as a block 121 thereof) from the second storage 292 to the lathe 610 for peeling the second log 120 or a block 121 receivable therefrom. Then the same lathe 610 can be used even if the qualities of the logs are different, because best peeling parameters can be determined from the primary information I11, I21.

However, an embodiment comprises transferring at least a part of the first log 110 (such as a block 111 thereof) from the first storage 291 to the lathe 610 for peeling the first log 110 or the first primary block 111 and transferring at least a part of the second log 120 (such as a block 121 thereof) from the second storage 292 to another lathe for peeling the second log 120 or a block 121 receivable therefrom.

The embodiment or another embodiment comprises conditioning the first log 110 for a first period of time and conditioning the second log 120 for a second period of time, wherein the second period of time lasts longer than the first period of time. This help to obtain suitable plasticity for logs of different quality.

The embodiment or another embodiment comprises conditioning the first log 110 at a first temperature and conditioning the second log 120 at a second temperature, wherein the second temperature is different from the first temperature. Also this help to obtain suitable plasticity for logs of different quality.

As indicated above, a log may be disposed. Thus, no veneer would be produced from that log. Such a log may be called a third log 130 (see Fig. 1). The log 130 of Fig. 1 shows both curvedness and a core with decay. Referring to Fig. 9, an embodiment comprises imaging a third log 130 to determine third primary information of the third log 130, the third primary information being indicative of at least one of the following: (i) information of knots in the third log, (ii) density profile of the third log, and (iii) shape of the third log. Since the quality of the third log 130 may be very low, e.g. the quality may be so low that the third log 130 cannot be peeled, the third primary information may be also indicative of at least one of the following: presence of a foreign object in the third log 130, and presence of decay in the third log 130. Herein presence means the fact of not being absent. Thus, information indicative of presence is also indicative on absence in the sense that these are mutually exclusive events. Then, the third primary information is used to determine that the third log 130 is to be disposed with, and, accordingly, the third log 130 is disposed.

As indicated above, it may be possible to pre-treat the third log 130 so that the remaining part of the third log can be peeled.

Preferably, the first model M1 and the third primary information of the third log are used to determine that the third log 130 is to be disposed with.

As indicated above, an embodiment of the method comprises controlling the debarking apparatus 410 using the first primary information of the first log 110. Referring to Fig. 8, typically, a debarking apparatus 410 comprises blades 430 for debarking the log 110 or the block 111, 112. Preferably, if the log 110 is cross-cut to the blocks 111, 112, the log 110 is debarked before cross-cutting, and correspondingly, the block 111 needs not be debarked. The blades 430 are pressed against the log 110 (or block 111) to be debarked. Therefore, a pressure of the debarking blades 430 acts on the log 110 (or block 111) while debarking. The debarking apparatus 410 typically comprises a subsystem 435 that is configured to control the pressure of the debarking blades 430 on the log 110 (or block 111). When debarking, the debarking blades 430 may be configured to rotate about an axis that is parallel to a longitudinal axis of the log 110 (or block 111) as indicated by arrows in Fig. 8. The longitudinal axis of the log 110 (or block 111) herein refers to a direction of length of the log 110 (or block 111). A longitudinal axis of the log 110 (or block 111) is surrounded by the annual rings of the log 110 (or block 111). Moreover, the debarking apparatus 410 comprises a feeder 420 for feeding the log 110 to be peeled to the debarking blades 430 of the debarking apparatus 410. The feeder 420 comprises typically a pair of rollers 422, which apply a pressure on the log 110 (or block 111) while feeding the log 110 (or block 111) to the blades 430.

Referring to Fig. 5a, in an embodiment the step of analysing 500 is done before debarking 400. In this case the accurate first primary information 111 concerning the first log 110 can be obtained from the first database 290 before debarking 400 the first log 110 (or block 111). Therefore, the debarking parameters can be optimized log-wise based on the first primary information I11. Thus, an embodiment of the method comprises controlling the debarking apparatus 410 using the first primary information I11 of the first log 110 by controlling at least one of
- a speed by which the first log 110 or the first primary block 111 is fed to the debarking blades of the debarking apparatus 410,
- a rotational speed of the debarking blades 430 for debarking the first log 110 or the first primary block 111,
- a pressure of the debarking blades 430 on the first log 110 or the first primary block 111, and
- a pressure of the feed rollers 422 on the first log 110 or the first primary block 111.

In this case, preferably, the method comprises controlling the debarking apparatus 410 before or while debarking the first log 110 or the first primary block 111 by using the first primary information I11 of the first log 110.

As an example, a first log 110 or the first primary block 111 comprising a soft surface part 112 (see Fig. 3b) may be debarked using a low pressure of the debarking blades 430 on the first log 110 (or block 111) and/or a low pressure of the feed rollers 422 on the first log 110 (or block 111). Thus, the debarking blades 430 and/or feed rollers 422 do not lower the quality of the sapwood of the log or block.

Furthermore, in case the step of analysing 500 reveals that debarking is not optimal, i.e. the logs are not sufficiently debarked or also some of the surface 112 of the logs is being debarked or the quality of the sapwood is diminished by the debarking, this information can be used in the control. For example, if the first or a second model is used to determine parameters for the debarking based on the first primary information 111, the first secondary information 112 may be used to teach the second model. In this way, the subsequent logs, e.g. a second log 120, can be more carefully debarked based on the primary information concerning the subsequent log. Therefore, an embodiment comprises
- using the first or a second model and the first primary information 111 of the first log 110 to determine debarking parameters for the debarking apparatus 410 for debarking the first log 110,
- updating (e.g. teaching) the first or the second model using the first secondary information 112 and the first primary information 111, and thereafter
- using the first or the second model and the second primary information 121 of the second log 120 to determine debarking parameters for the debarking apparatus 410 for debarking the second log 120.

The debarking parameters are also applied in the debarking apparatus 410. In the embodiment, the first secondary information 112 is also indicative of successfulness of the debarking 400.

Updating the first or the second model may be done as known in the field of numerical modelling or artificial intelligence. The first or the second model may be a data-driven model. The first or the second model may be a neural network model. The first or the second model may be updated, even if the step of analysing 500 the first log 110 is only after the step of debarking 400. The first or the second model is created before it is used.

However, referring to Fig. 5b, in an embodiment the step of analysing 500 is done after debarking 400. In this case the accurate first primary information 111 concerning the first log 110 is obtained from the first database 290 after debarking 400 the first log 110 (or block 111). However, the first secondary information 112, which is obtained by the analysing 500, may contain information indicative of the successfulness of the debarking 400. Thus, by comparing the first primary information 111 and first secondary information 112, the debarking apparatus can be controlled for better debarking another log or block, e.g. a second log 120 or block 121 receivable therefrom. The controlling may be done using the first or the second model, as discussed above. Thus, an embodiment of the method comprises controlling the debarking apparatus 410 using the first primary information 111 of the first log 110 by controlling at least one of
- a speed by which a log or the block receivable therefrom by cross-cutting is fed to the debarking blades of the debarking apparatus 410,
- a rotational speed of the debarking blades 430 for debarking a log or a block receivable therefrom by cross-cutting,
- a pressure of the debarking blades 430 on a log or a block receivable therefrom by cross-cutting, and
- a pressure of the feed rollers 422 on a log or a block receivable therefrom by cross-cutting.

In any case, it is possible to monitor at least one of the following:
- a condition (e.g. need for maintenance) of the debarking blades 430 of the debarking apparatus 410,
- a need for control the pressure of the debarking blades 430 on a log or a block receivable therefrom by cross-cutting,
- a condition of the subsystem 435 that is configured to control the pressure of the debarking blades 430 on the log or a block receivable therefrom by cross-cutting,
- a condition (e.g. need for maintenance) of the feeder(s) 420, 422 of the debarking apparatus 410,
- a pressure of the feeder(s) 420, 422 of the debarking apparatus 410.

Such monitoring may be done from the first secondary information 112, at least when the first secondary information 112 is indicative of the successfulness of the debarking 400.

As indicated above, an embodiment of the method comprises controlling the lathe 610 using the first primary information 111 of the first log 110. In particular, the lathe 610 may be controlled log-wise (or block-wise) using the information 111. Thus, a preferable embodiment comprises before or while peeling the first log 110 or the first primary block 111, controlling the lathe 610 using the first primary information 111 of the first log 110. Figures 7a and 7b show components of typical lathe 610 as well as peeling the first log 110 or block 111 to form the veneer 101. Fig. 7a is an end view, as seen in the longitudinal direction of the first log 110 or block 111. Fig. 7b is a top view, as seen from above. The arrows in Fig. 7b indicate direction of rotation and/or direction of the veneer 101.

Referring to Figs. 7a and 7b, typically a lathe 610 comprises two spindles 630 in between which the log 110 (or block 111) is arranged when being peeled. The spindles 630 are pressed against ends of the log 110 (or block 111) so as to grip the log 110 (or block 111). When at least one of the spindles is rotated, also the log 110 (or block 111) is rotated. However, spindle-free lathes are also known. Therein the log 110 (or block 111) is rotated only by rollers 632 that press the log from its peripheral surface. Even if the lathe 610 comprises a spindle 630, the lathe 610 may comprise a roller 632 configured to press the log 110 (or block 111) from its peripheral surface to stabilize the log 110 (or block 111) while peeling. The roller 632 may be e.g. a back up roll (BUR) of the lathe.

The lathe 610 comprises a knife 620 for peeling the veneer 101 from the log 110 (or block 111). The lathe 610 comprises a nose bar 624. While peeling, the nose bar 624 is pressed against a surface of the log 110 (or block 111) so that a gap 625 remains between the nose bar 624 and the knife 620. As well known, the nose bar 624 may be of a rotatory type or a stationary type. The veneer 101 exits through the gap 625. The gap 625 determines, among other things, a thickness of the veneer 101 (and veneer 102 when a second log 120 or block 121 is peeled). An angle α of the knife 620 affects the quality of the veneers. The angle α of the knife 620 may be defined as indicated in Fig. 7a. As the knife 620 is turned, the angle α of the knife 620 changes.

To increase yield and/or quality of the veneer 101, the angle α of the knife 620 may be controlled e.g. based on density of the log 110 (or block 111). Thus, the angle α of the knife may be controlled before peeling the first log 110 (or block 111). However, the density of the log 110 (or block 111) needs not be constant, whereby the angle α of the knife may be controlled while peeling the first log 110 (or block 111).

Peeling of the log 110 (or block 111) conventionally is done such that a peripheral velocity of the log 110 (or block 111) remains constant during peeling. Moreover, during peeling, the radius of the log 110 (or block 111) decreases because veneer 101 is produced. In effect, conventionally, the angular speed of the spindles 630 and/or the rollers 632 is increased during peeling so that the peripheral speed of the log 110 (or block 111) remains constant. However, based on the first primary information 111, the peripheral speed of the first log 110 (or block 111) may be controlled.

Moreover, conventionally, the log 110 (or block 111) is peeled to such an extent that a minimum radius of the log 110 (or block 111) is reached, wherein the minimum radius is the same for all logs (or blocks 111). Then the peeling is stopped, and e.g. the log 110 (or block 111) is moved away from the knife 620 to stop peeling thereof. Another log (or block) is peeled thereafter. To clarify, Fig. 4a shows a log 110 from which veneer 101 is peeled. Fig. 4b shows a remainder 119, i.e. a remaining log, after the log 110 has been peeled. Conventionally, a log is peeled until a minimum radius for the remainder 119 is reached. Such a minimum radius may be e.g. around 3 to 7 cm. However, based on the first primary information 111, the peeling may be stopped earlier or later, depending on the quality of the core of the log that is being peeled.

Thus, an embodiment of the method comprises before or while peeling the first log 110 or the first primary block 111, controlling the lathe 610 using the first primary information 111 of the first log 110 by controlling at least one of
- an angle α of a knife of the lathe 610,
- a peripheral speed for peeling the first log 110 or the first primary block 111,
- a knife gap 625 of the lathe 610,
- a pressure that the nose bar 624 imposes on the first log 110 or the first primary block 111,
- a pressure that a spindle 630 imposes on the first log 110 or the first primary block 111,
- a location of a centre of a spindle 630 relative to a centre of the first log 110 or the first primary block 111,
- a pressure of a roller 632 applied on a surface of the first log 110 or the first primary block 111, and
- a diameter of the first log 110 or the first primary block 111 after peeling.

As for the angle α of a knife of the lathe 610, it has been noticed that for a hard log, the angle α needs to be large. Typically, a density of wood is indicative of hardness of the wood. Therefore, an embodiment comprises determining form the first primary information 111 that a hardness of the first log 110 or the first primary block 111 is higher than a hardness of a log (or block) previously peeled, and as a result of said determining, increasing the angle α of the knife 620 of the lathe. The angle α of the knife 620 of the lathe 610 may be controlled e.g. before starting to peel the first log 110 or the first primary block 111. In addition or alternatively, the angle α of the knife 620 may be controlled while peeling the first log 110 or the first primary block 111. Therefore, an embodiment comprises determining form the first primary information 111 that a hardness of a core part the first log 110 or the first primary block 111 is higher than a hardness of a surface part of the first log 110 or the first primary block 111, and as a result of said determining, after peeling a surface part of the first log 110 or the first primary block 111 and before peeling a core part of the first log or the first primary block, increasing the angle α of the knife 620 of the lathe. In addition or as an alternative to increasing the angle α, a pressure that the nose bar 624 imposes on the first log 110 or the first primary block 111 can be decreased.

As for controlling the peripheral speed for peeling the first log 110 or the first primary block 111, the primary information 111 may be indicative of at least one of
- exceptionally many knots in the first log 110 or the first primary block 111,
- soft and/or rotten core of the first log 110 or the first primary block 111, and
- a high density of the wood material of the first log 110 or the first primary block 111.

Each one of these typically imply that a quality of the veneer 101 can be improved by slowing down the peeling velocity, in particular the peripheral velocity of the first log 110 or the first primary block 111. Thus, an embodiment comprises, determining at least one of the these properties from the first primary information; and as a result, decreasing a peripheral speed of the log (or block) to be peeled or the log (or block) that is peeled. The peripheral speed for the first log (or the first primary block) may be controlled before peeling, since the target for the peripheral speed is a process parameter even before the first log 110 or the first primary block 111 actually rotates. The peripheral speed may be decreased also while peeling the first log (or the first primary block). Having exceptionally many knots may involve determining that a number of knots exceeds a threshold. Having a high density may involve determining that a density exceeds a threshold.

As for controlling a knife gap 625 of the lathe 610, the first primary information may be indicative of exceptionally light wood and/or exceptionally wet wood. It is known that such wood shrinks, i.e. becomes thinner, during drying. Therefore, to compensate for the drying-induced thinning of the veneer, the knife gap 625 can be increased as a result of determining, from the first primary information 111, that a density of the wood material of the first log 110 or the first primary block 111 is less than a threshold and/or a moisture content of the wood material of the first log 110 or the first primary block 111 is more than a threshold.

As for a pressure that a spindle 630 imposes on the first log 110 or the first primary block 111, the pressure may be optimized in view of the hardness or density of the core material of the log or block. As for a location of a centre of a spindle 630 relative to a centre of the first log 110 or the first primary block 111, the first log 110 or the first primary block 111 may be centred with respect to the spindle(s) 630. However, if a shape of the first log 110 or the first primary block 111 is irregular, the yield of veneer may be higher when the first log 110 or the first primary block 111 is not centred. The first log 110 or the first primary block 111 may be positioned relative to the spindle(s) 630 so as to maximize the yield.

As for a pressure of a roller 632, e.g. a back up roll (BUR), applied on a surface of the first log 110 or the first primary block 111, too high a pressure may increase energy consumption of the process. However a higher pressure may be applied to slightly straighten the log 110 or block 111 and in this way decrease the curvedness of the log 110 or block 111 for peeling. Moreover, a roller 632 may be used to compensate for the pressure of the knife of the lathe, in this way allowing to peel the log or block to a smaller diameter (cf. below). How the roller 632 presses the log or block may be controlled by controlling a position of the roller 632 relative to the log or the block or a pressure that the roller 632 applies on the log or block. A lathe 610 may comprise more than one roller 632, which improves stabilisation of the log 110 or block 111 during peeling.

As for a minimum diameter of the first log 110 or block 111 after peeling, as detailed above, peeling is stopped once the minimum radius is reached. However, based on the first primary information 111 it may be possible to determine that the core of the first log 110 or block 111 is exceptional (very hard or very soft) so that core should not be used for production of veneer. Then, the minimum radius may be increased accordingly. As a result, when the knife 620 of the lathe 610 reaches the core of the first log 110 or the first primary block 111, the peeling can be stopped. The minimum radius can be set before or while peeling the first log 110 or the first primary block 111. In the alternative the primary information 111 may be indicative of also the core 111 comprising peelable material, whereby the minimum radius can be decreased.

Indicating some of these aspects in terms of wood properties, the following control procedures can be used:
- softer wood:
   ∘ decrease the angle α of the knife 620 of the lathe 610,
   ∘ increase a pressure that the nose bar 624 imposes on the first log 110 or first primary block 111, or
   ∘ decrease a pressure that the spindle imposes 620 on the first log 110 or first primary block 111.
- harder wood:
   ∘ increase the angle α of the knife 620 of the lathe 610,
   ∘ decrease a pressure that the nose bar 624 imposes on the first log 110 or first primary block 111, or
   ∘ increase a pressure that the spindle imposes 620 on the first log 110 or first primary block 111.
- softer heartwood:
   ∘ decrease the peripheral speed of the first log 110 or first primary block 111,
   ∘ decrease a pressure that the spindle imposes 620 on the first log 110 or first primary block 111, or
   ∘ stop peeling in a controlled manner (i.e. controlling the minimum radius for the remainder of the first log).
- harder heartwood:
   ∘ increase a pressure that the spindle imposes 620 on the first log 110 or first primary block 111, or
   ∘ decrease a pressure that the nose bar 624 imposes on the first log 110 or first primary block 111.
- more knots in the first log:
   ∘ increase a pressure that the spindle imposes 620 on the first log 110 or first primary block 111,
   ∘ decrease the peripheral speed of the first log 110 or first primary block 111, or
   ∘ increase the angle α of the knife 620 of the lathe 610.

The parameters of the lathe 610 can be adjusted so as to optimize the yield of the process. The yield refers to a mass percentage of the veneer 101 with respect to a mass of the first log 110. Optimizing the yield may involve maximizing the yield. Optimizing the yield may involve maximizing the yield of such veneer, of which quality is sufficiently high. The parameters of the lathe 610 can be adjusted so as to maximize the yield of the process. However, optimizing the yield may involve maximizing the yield on the condition that a certain productivity is achieved. The productivity herein refers to an amount of the produced veneer during a unit time, typically a day. The productivity can be affected, in addition to improving yield, by disposing low-quality logs before peeling (e.g. before conditioning or after the analysing 500) because peeling only high quality logs improves the throughput of the lathe(s).

As another example, discarding e.g. an exceptional core of a log inevitably decreases the yield to some extent. However, it may increase the productivity, as exceptional material can typically only be peeled with slower peripheral speed. Moreover, not peeling the core may result if fever manufacturing stops and lesser maintenance. In terms of manufacturing costs the yield and productivity needs to be balanced based on e.g. price of raw material, price of product, and commitments related to deliveries.

The lathe 610 can be controlled by an operator (human) or using a third model (or the first or the second model). The third model (or the first or the second model) is configured to receive, as an input, the at least such a part of the first primary information 111 that concerns the first block (111 or 112) or first log 110 that is being peeled or going to be peeled; and to give, as an output, proper peeling parameters or changes needed to the peeling parameters. It is possible that only one model is configured to determine, from the first primary information 111, all of: a proper class of the first log 110, proper debarking parameters (or a needed change thereto), and proper peeling parameters (or a needed change thereto). In the alternative, different models may be used for different purposes.

As detailed above, an embodiment comprises after peeling 600 the first log 110 or the first primary block 111, imaging 700 at least a part of the veneer 101 to obtain first tertiary information 113 indicative of an amount of veneer 101 manufacturable from the first log 110. The first tertiary information 113 can be used to create or update the third model (or the first or the second model). In particular the (third) model or that part of the (first or second) model that is used give, as an output, proper peeling parameters or changes needed to the peeling parameters.

Moreover, the third model (or the first or the second model) can be used to determine parameters for controlling the lathe 610. Thus, an embodiment comprises using a first, a second, or a third model and the first primary information 111 of the first log 110 to obtain peeling parameters or a change to the peeling parameters and controlling the lathe by using the peeling parameters or by updating the peeling parameters by using the change to the peeling parameters. Thus, the first primary information 111 of the first log 110 can fed to the third model (or the first or the second model), which then outputs proper peeling parameters of a needed change to the peeling parameters. These parameters or their change can then be used with the lathe. In case only a part of the log 110 (the part being a cross-cut block) is peeled, in addition to the first primary information I11, information indicative of which part (i.e. which block 111, 112) of the first log 110 is being peeled can be sent to the third model (or the first or the second model). As indicated above, preferably reasonable accurate information should be obtained in the step of imaging 200 the first log 110 (and also imaging the second log 120 and/or imaging the third log 130) in order to be able to determine the proper parameters reliably. As for imaging 200 the logs, in particular imaging the first log 110 the first time, in an embodiment, the method comprises at least one of (i) forming an X-ray image of the first log 110, (ii) forming an image of the first log 110 using ultrasound transducer or ultrasound transducers, (iii) forming an image of the first log 110 using magnetic resonance (NMR), and (iv) forming an image of the first log 110 using beta-radiation. All these methods are capable of imaging at least the inner part of the first log 110, wherein the inner part of the first log is arranged beneath the surface of the first log 110. The first primary information 111 is also determined before debarking.

Preferably, forming an X-ray image of the first log 110 comprises imaging the first log 110 using X-ray from at least a first direction. However, at least for the time being, a more accurate three-dimensional image of the log can be obtained by imaging from two different directions. Thus, preferably, forming an X-ray image of the first log 110 (and also the step of imaging 200 the first log 110) comprises imaging the first log 110 using X-ray from a first direction and from a second direction, the second direction being different from the first direction. This has been found to produce reliable results without use of excessively expensive devices. Referring the Fig. 2, the first X-ray image of the first log 110 may be formed by using a first X-ray source 210 in combination with a first X-ray detector 212 and a second X-ray source 220 in combination with a second X-ray detector 222. While imaging the log 110 a part of the first log 110 is arranged between the first X-ray source 210 and the first X-ray detector 212; and a part of the first log 110 is arranged between the second X-ray source 220 and the second X-ray detector 222. When taking the image of the whole first log 110, it may be moved in the longitudinal direction so as to scan all parts of the first log 110. As an example, the method and device disclosed in US 6,157,698 may be used for the purpose. Hereinabove the term X-ray refers to electromagnetic waves of high energy and very short wavelength, which is able to pass through many materials opaque to light. Typically X-rays have a wavelength in the range of 0.01-10 nm.

One way of forming the X-ray image from two different directions is to have only one X-ray source that is configured to rotate about the log while imaging the log. In this way, the log becomes imaged from multiple directions, including a first and a second direction.

In an embodiment, the image is formed such that the first log moves relative to the X-ray detectors while forming the first X-ray image. The first log may move relative to the ground and/or the X-ray detector(s) or source(s) may move relative to the ground.

The image obtained by X-ray may be supplemented with other shape-related data. Therefore, in an embodiment, the imaging 200 the first log for the first time further comprises scanning the first log from a third direction using a third laser scanner. More preferably, the imaging 200 further comprises scanning the first log 110 from a fourth direction using a fourth laser scanner. Preferably, the first log 110 is scanned from at least three, preferably four, different direction using laser scanners. In this way a shape of a surface of the first log can be accurately obtained. More preferably, the imaging 200 further comprises imaging the first log 110 using a first camera. Herein a "camera" refers to a device configured to capture an image of light reflected from a surface of the first log 110. Even more preferably, the imaging 200 comprises imaging the first log 110 using two cameras. Two cameras, instead of only one, are capable of obtaining three-dimensional information of the log 110.

What has been said about imaging 200 the first log 110 applies to imaging a second log 120 *mutatis mutandis.* What has been said about imaging 200 the first log 110 applies to imaging a third log 130 *mutatis mutandis.* Reference is made to Figs. 5c and 9.

As indicated above, not so accurate information needs to be obtained in the step of analysing 500 the first log 110 or the first primary block 111. As for analysing 500 the logs or blocks, in particular analysing 500 the first log 110 or the first primary block 111 the second time, as indicated above, it may suffice to read an information carrier or an information marker provided to the first log 110 or the first primary block 111. However, if an information carrier/marker is not used, the analysing 500 the first log 110 or the first primary block 111 the second time comprises, in an embodiment, at least one of the following:
- scanning the first log 110 or the first primary block 111 from a primary direction using a first laser scanner and scanning the first log 110 or the first primary block 111 from a secondary direction using a second laser scanner, wherein the primary direction is different from the secondary direction,
- imaging the first log 110 or the first primary block 111 using a second camera (or at least two cameras), and
- forming a second X-ray image of the first log 110 or the first primary block 111.

In the step of analysing 500, the first log 110 or the first primary block 111 may be scanned from at least three, preferably four, different directions using laser scanners. In this way a three-dimensional shape of a surface of the first log can be accurately obtained. Preferably, as many laser scanners are used in the step of imaging 200 the log 110 as in the step of analysing 500 the log 110 or the block 111.

It has been found that the first primary information I11 can reliably be associated with the first secondary information I12, when the analysing 500 the first log 110 or the first primary block 111 the second time comprises form ing a second X-ray image of the first log 110 or the first primary block 111, respectively. However, the step of analysing 500 needs not comprise forming an X-ray image from different directions.

The first primary and first secondary information I11, I12 can be associated with each other even more reliably, when the analysing 500 the first log 110 or the first primary block 111 the second time comprises all of (i) forming a second X-ray image of the first log 110 or the first primary block 111, scanning the first log 110 or the first primary block 111 from a primary direction using a first laser scanner and scanning the first log 110 or the first primary block 111 from a secondary direction using a second laser scanner, wherein the primary direction is different from the secondary direction, and (iii) imaging the first log 110 or the first primary block 111 using a second camera.

What has been said about analysing 500 the first primary block 111 applies to analysing first secondary block 112 *mutatis mutandis.* What has been said about analysing 500 the first log 110 or the first primary block 111 applies to analysing a second log 120 or a block (121, 122) manufacturable therefrom *mutatis mutandis.* What has been said about analysing 500 the first log 110 or the first primary block 111 applies to analysing a third log 130 or a block manufacturable therefrom *mutatis mutandis.*

## Claims

1. A method for manufacturing veneer, the method comprising
- conditioning a first log or a first primary block cross-cut from the first log,
- debarking the first log or the first primary block using a debarking apparatus,
- peeling the first log or the first primary block using a lathe to form the veneer,
- before conditioning, debarking, and peeling the first log or the first primary block, imaging the first log a first time to determine first primary information of the first log, the first primary information being indicative of at least one of the following: (i) information of knots in the first log, (ii) density profile of the first log, and (iii) shape of the first log,
- storing the first primary information in a first database,
- after imaging the first log the first time and before peeling the first log or the first primary block, analysing the first log or the first primary block a second time to determine first secondary information,
- accessing the first database with the first secondary information to receive the first primary information from the first database, and
- controlling the debarking apparatus and/or the lathe using the first primary information of the first log.

2. The method of claim 1 comprising
- before or while peeling the first log or the first primary block, controlling the lathe using the first primary information of the first log and/or
- imaging a surface of the first log and an inner part of the first log, the inner part being arranged beneath the surface of the first log, to determine the first primary information of the first log.

3. The method of claim 1 or 2, comprising
- after imaging the first log the first time and before the conditioning, the debarking, and the peeling the first log or the first primary block, using the first primary information of the first log to classify the first log such that the first log belongs to a first class of logs and does not belong to a second class of logs, and
- transferring the first log to a first storage that comprises only logs that belong to the first class of logs;
preferably the method comprises
- using a first model and the first primary information of the first log to classify the first log such that the first log belongs to the first class of logs and does not belong to the second class of logs;
more preferably,
- the first primary information is also indicative of at least one of: (iv) absence of foreign objects from the first log and (v) absence of decay from the first log.

4. The method of any of the claims 1 to 3, comprising
- after peeling the first log or the first primary block to form the veneer, imaging the veneer to obtain first tertiary information of the veneer and
[A]
- creating a first model using the first tertiary information and the first primary information, the first model being configured to classify a second log based on second primary information such that the second log belongs to a first class of logs and does not belong to a second class of logs, wherein the second primary information is indicative of at least one of the following: (i) information of knots in a second log, (ii) density profile of the second log, and (iii) shape of the second log and/or
[B]
- before imaging the veneer, using a first model and the first primary information of the first log to classify the first log such that the first log belongs to the first class of logs and does not belong to the second class of logs, and
- after imaging the veneer, updating, e.g. teaching, the first model using the first tertiary information and the first primary information.

5. The method of claims 1 to 4, comprising
- after imaging the first log the first time, cross-cutting the first log to obtain the first primary block and a first secondary block,
- peeling the first primary block to form veneer and
- analysing the first log to determine first secondary information or analysing the first primary block to determine first secondary information;
preferably the method comprises
- analysing the first primary block to determine the first secondary information; preferably, the method comprises
- peeling also the first secondary block to produce veneer;
optionally the method comprises
- disposing the first secondary block.

6. The method of claims 1 to 5, comprising
- after imaging the first log the first time, imaging a second log a first time to determine second primary information of the second log, the second primary information being indicative of at least one of the following: (i) information of knots in the second log, (ii) density profile of the second log, and (iii) shape of the second log, and
- storing the second primary information in the first database;
preferably the method comprises
- storing the second primary information in the first database before accessing the first database with the first secondary information to receive the first primary information from the first database;
more preferably the method comprises
- after imaging the second log a first time and before peeling the second log or a second primary block cross-cut from the second log, analysing the second log or a second primary block cross-cut from the second log a second time to determine second secondary information,
- accessing the first database with the second secondary information to receive the second primary information from the first database, and
- controlling the debarking apparatus that debarks the second log or the second primary block using the second primary information and/or controlling the lathe that peels the second log or the second primary block using the second primary information.

7. The method of claim 6, comprising
- using the first primary information of the first log and a first or a second model to determine debarking parameters for the debarking apparatus for debarking the first log or the first primary block,
- updating the first or the second model using the first secondary information and the first primary information, and thereafter
- using the first or the second model and the second primary information to determine debarking parameters for the debarking apparatus for debarking the second log or the second primary block.

8. The method of claim 6 or 7, comprising
- after imaging the second log the first time and before the conditioning, the debarking, and the peeling the second log or the second primary block, using the second primary information of the second log to classify the second log such that the second log belongs to a second class of logs and does not belong to the first class of logs, and
- transferring the second log to a second storage that comprises only logs that belong to the second class of logs;
preferably the method comprises
- using a first model and the second primary information of the second log to classify the second log such that the second log belongs to the second class of logs and does not belong to the first class of logs;
more preferably,
- the second primary information is also indicative of at least one of: (iv) absence of foreign objects from the second log and (v) absence of decay from the second log.

9. The method of any of the claims 6 to 8, comprising
[A]
- transferring at least a part of the first log from the first storage to the lathe for peeling the first log or the first primary block and
- transferring at least a part of the second log from a/the second storage to another lathe for peeling the second log or the second primary block and/or
[B]
- conditioning by soaking the first log or the first primary block for a first period of time and
- conditioning by soaking the second log or the second primary block for a second period of time, wherein
- the second period of time lasts longer than the first period of time and/or
[C]
- conditioning by steaming the first log or the first primary block for a first period of time and
- conditioning by steaming the second log or the second primary block for a second period of time, wherein
- the second period of time lasts longer than the first period of time and/or
[D]
- conditioning the first log or the first primary block at a first temperature and
- conditioning the second log or the second primary block at a second temperature, wherein
- the second temperature is different from the first temperature.

10. The method of any of the claims 1 to 9, comprising
- imaging a third log to determine third primary information of the third log, the third primary information being indicative of at least one of the following: (i) information of knots in the third log, (ii) density profile of the third log, and (iii) shape of the third log,
- using the third primary information of the third log to determine that the third log is to be disposed with or to be pre-treated, and
- disposing or pre-treating the third log;
in an embodiment
- the third primary information is also indicative of at least one of (iv) presence of a foreign object in the first log, and (v) presence of decay in the first log.

11. The method of any of the claims 1 to 10, comprising
- before the conditioning, the debarking, and the peeling the first log or the first primary block, attaching an information carrier to the first log and/or printing an information marking to the first log or the information carrier, wherein
- analysing the first log or the first primary block the second time comprises reading the information carrier or the information marking, and
- using the information on the information carrier or the information marking for receiving the first primary information from the first database.

12. The method of any of the claims 1 to 11, wherein
- analysing the first log or the first primary block the second time comprises imaging the first log or the first primary block to receive a second image of the first log or the first primary block and
- using the second image for receiving the first primary information from the first database;
preferably, analysing the first log or the first primary block the second time comprises
- imaging the first log or the first primary block using X-ray;
more preferably, analysing the first log or the first primary block the second time comprises
- imaging the first log or the first primary block using X-ray, and
- scanning the first log or the first primary block from a primary direction using a first laser scanner and scanning the first log or the first primary block from a secondary direction using a second laser scanner, wherein the primary direction is different from the secondary direction;
optionally the method further comprises
- imaging the first log or the first primary block using a second camera.

13. The method of any of the claims 1 to 12, wherein the imaging the first log the first time comprises at least one of
- imaging the first log using X-ray,
- imaging the first log using an ultrasound transducer or ultrasound transducers,
- imaging the first log using magnetic resonance (NMR), and
- imaging the first log using beta-radiation;
preferably, the method comprises
- imaging the first log from a first direction using X-ray and from a second direction using X-ray, the second direction being different from the first direction,
more preferably, the method comprises
- imaging the first log from a first direction using X-ray and from a second direction using X-ray, the second direction being different from the first direction, such that the first log moves relative to an X-ray detector while imaging the first log.

14. The method of any of the claims 1 to 13, wherein the imaging the first log the first time comprises at least one of
- scanning the first log from a third direction using a laser scanner,
- scanning the first log from a third direction and a fourth direction, which is different from the third direction, using laser scanners, and
- imaging the first log using a first camera or at least two cameras.

15. The method of any of the claims 1 to 14, wherein analysing the first log or the first primary block a second time comprises at least one of
- scanning the first log or the first primary block from a primary direction using a first laser scanner,
- imaging the first log or the first primary block using a second camera or at least two cameras,
- imaging the first log or the first primary block using X-ray, and
- reading an information carrier provided to the first log or the first primary block;
preferably, analysing the first log or the first primary block a second time comprises
- imaging the first log or the first primary block using X-ray;
preferably, analysing the first log or the first primary block a second time comprises
- imaging the first log or the first primary block using X-ray, and at least one of
- (a) scanning the first log or the first primary block from a primary direction using a first laser scanner and scanning the first log or the first primary block from a secondary direction using a second laser scanner, wherein the primary direction is different from the secondary direction, and
- (b) imaging the first log or the first primary block using a second camera or at least two cameras.

16. The method of any of the claims 1 to 15, comprising before or while peeling the first log or the first primary block, controlling the lathe by controlling, using the first primary information of the first log, at least one of
- an angle of a knife of the lathe,
- a peripheral speed for peeling the first log or the first primary block,
- a knife gap of the lathe,
- a pressure that a nose bar of the lathe imposes on the first log or the first primary block,
- a pressure that a spindle imposes on the first log or the first primary block,
- a location of a centre of a spindle relative to a centre of the first log or the first primary block,
- a pressure or a position of a roller applied on a surface of the first log or the first primary block, and
- a diameter of the first log or the first primary block after peeling;
preferably the method comprises before or while peeling the first log or the first primary block, controlling the lathe by controlling, using the first primary information of the first log, at least one of
- an angle of a knife of the lathe,
- a peripheral speed for peeling the first log or the first primary block,
- a knife gap of the lathe, and
- a pressure that a spindle imposes on the first log or the first primary block, preferably, the method comprises while peeling the first log or the first primary block,
controlling, using the first primary information of the first log, at least one of
- an angle of a knife of the lathe,
- a pressure that a nose bar imposes on the first log or the first primary block,
- a peripheral speed of the first log or the first primary block that is being peeled,
- a knife gap of the lathe,
- a pressure that a spindle of the lathe imposes on the first log or the first primary block, and
- a minimum diameter of the first log or the first primary block after peeling.

17. The method of any of the claims 1 to 16, comprising
- after peeling the first log or the first primary block to form the veneer, imaging the veneer to obtain first tertiary information of the veneer and
[A]
- creating a first, a second, or a third model using the first tertiary information and the first primary information, the model being configured to receive, as an input, at least such a part of the first primary information 111 that concerns the first log or the first primary block that is being peeled or going to be peeled, and to give, as an output, peeling parameters or a change to the peeling parameters and/or
[B]
- updating, such as teaching, a first, a second, or a third model using the first tertiary information and the first primary information, the model being configured to receive, as an input, at least such a part of the first primary information that concerns the first log or the first primary block that is being peeled going to be peeled, and to give, as an output, peeling parameters or a change to the peeling parameters and/or
[C]
- before imaging the veneer, using a first, a second, or a third model and the first primary information of the first log to obtain peeling parameters or a change to the peeling parameters and
- controlling the lathe by using the peeling parameters or by updating the peeling parameters by using the change to the peeling parameters.

18. The method of any of the claims 1 to 17, comprising controlling the debarking apparatus using the first primary information of the first log by controlling at least one of
- a speed by which the first log or the first primary block is fed to the debarking blades of the debarking apparatus,
- a speed by which a log or a block is fed to the debarking blades of the debarking apparatus,
- a rotational speed of the debarking blades for debarking the first log or the first primary block,
- a rotational speed of the debarking blades,
- a pressure of the debarking blades on the first log or the first primary block,
- a pressure of the debarking blades on a peeled log or a peeled block,
- a pressure of the feed rollers on the first log or the first primary block, and
- a pressure of the feed rollers on a peeled log or a peeled block;
preferably, the method comprises
- before peeling the first log or the first primary block, analysing the first log or the first primary block a second time to determine first secondary information, accessing the first database with the first secondary information to receive the first primary information from the first database, and controlling the debarking apparatus before or while debarking the first log or the first primary block using the first primary information of the first log by controlling at least one of
- a speed by which the first log or the first primary block is fed to the debarking blades of the debarking apparatus,
- a rotational speed of the debarking blades for debarking the first log or the first primary block,
- a pressure of the debarking blades on the first log or the first primary block, and
- a pressure of the feed rollers on the first log or the first primary block.

19. The method of any of the claims 1 to 18, wherein
- the first primary information is indicative of (i) information of knots in the first log, (ii) density profile of the first log, (iii) shape of the first log, (iv) absence of foreign objects from the first log, and (v) absence of decay from the first log, and/or
- the first primary information is indicative of at least one or all of:
- a length of the first log,
- a diameter of the first log,
- a diameter of the first log as a function of position from an end of the first log,
- a shape of the first log,
- a conicity of the first log,
- an ellipticity of the first log,
- a multiple crookedness of the first log,
- dimensions of the knots of the of the first log,
- a number density of the knots of the of the first log,
- densities of the knots of the first log,
- locations of the knots of the of the first log,
- directions of the knots of the of the first log,
- types of the knots of the of the first log,
- location(s) of low-quality wood in the of the first log,
- presence or amount of rot in the of the first log,
- presence of cracks in the first log,
- proportion of sapwood and core wood of the first log,
- a peripheral area of the first log,
- a thickness or amount of bark of the first log,
- thicknesses of tree rings in the first log, and
- a volume of the first log.
